# EUROPEAN PATENT SPECIFICATION

(11) **EP 1 461 426 B1**
(45) Date of publication and mention of the grant of the patent: **09.01.2008**
(21) Application number: 02787465.0
(22) Date of filing: 20.12.2002
(51) Int. Cl.: C12N 9/12

(54) **EUBACTERIAL RNA-POLYMERASE MUTANTS FOR INCREASING HETEROLOGOUS GENE EXPRESSION**
MUTANE EUBAKTERIELLE RNA POLYMERASE FÜR DIE ERHÖHUNG HETEROLOGER GENEXPRESSION
MUTANTS DE L' ARN POLYMERASE D'EUBACTERIE POUR AUGMENTER L'EXPRESSION DE GENES HETEROLOGUES

(30) Priority: 29.12.2001 DK 200101972; 21.02.2002 DK 200200274
(43) Date of publication of application: 29.09.2004
(73) Proprietor: Novozymes A/S, 2880 Bagsvaerd (DK)
(72) Inventor: JORGENSEN, Steen, Troels, DK-3450 Allerod (DK); ANDERSEN, Jens, Toenne, DK-2850 Naerum (DK); BANKE, Niels, DK-2860 Soborg (DK); NIELSEN, Preben, DK-2970 Horsholm (DK)
(86) International application number: PCT/DK2002/000886
(87) International publication number: WO 2003/055996

(56) References cited:
- EP-A- 1 239 040
- WO-A-01/90393
- INGHAM C J ET AL: "Mutations in the beta subunit of the Bacillus subtilis RNA polymerase that confer both rifampicin resistance and hypersensitivity to NusG." MICROBIOLOGY (READING), vol. 146, no. 12, December 2000 (2000-12), pages 3041-3049, XP002236289 ISSN: 1350-0872
- NICHOLSON WAYNE L ET AL: "The spectrum of spontaneous rifampin resistance mutations in the rpoB gene of Bacillus subtilis 168 spores differs from that of vegetative cells and resembles that of Mycobacterium tuberculosis." JOURNAL OF BACTERIOLOGY, vol. 184, no. 17, September 2002 (2002-09), pages 4936-4940, XP002236290 September, 2002 ISSN: 0021-9193
- YANG XIAOFENG ET AL: "Streptolydigin resistance can be conferred by alterations to either the beta or beta' subunits of Bacillus subtilis RNA polymerase." JOURNAL OF BIOLOGICAL CHEMISTRY, vol. 270, no. 41, 1995, pages 23930-23933, XP002236291 ISSN: 0021-9258
- DATABASE BIOSIS [Online] BIOSCIENCES INFORMATION SERVICE, PHILADELPHIA, PA, US; 1979 KANE J F ET AL: "INCREASED LEVELS OF DI HYDRO FOLATE REDUCTASE IN RIFAMPIN RESISTANT MUTANTS OF BACILLUS-SUBTILIS" Database accession no. PREV197968008851 XP002236292 & JOURNAL OF BACTERIOLOGY, vol. 137, no. 2, 1979, pages 1028-1030, EN ISSN: 0021-9193
- DATABASE BIOSIS [Online] BIOSCIENCES INFORMATION SERVICE, PHILADELPHIA, PA, US; 1991 HU X ET AL: "STUDIES ON THE SCREENING OF THERMOSTABLE ALPHA AMYLASE-PRODUCING STRAINS" Database accession no. PREV199293039721 XP002236293 & ACTA MICROBIOLOGICA SINICA, vol. 31, no. 4, 1991, pages 268-273, ISSN: 0001-6209

## Description

### FIELD OF INVENTION

The present invention relates to an isolated mutant *Bacillus* bacterium comprising at least one mutation resulting in a substitution of at least one amino acid in the beta-subunit of the RNA-polymerase encoded for by the *rpoB*-gene providing an altered production of a product of interest when said production of a product of interest is compared to the production of the same product in an isogenic parent strain grown at identical conditions. Another aspect of the invention relates to a process for producing at least one product of interest in a mutant and to a use of the mutant according to the invention for producing at least one product of interest.

### BACKGROUND OF THE INVENTION

In the industrial production of polypeptides it is of interest to achieve a product yield as high as possible. One way to increase the yield is to increase the copy number of a gene encoding a polypeptide of interest. This can be done by placing the gene on a high copy number plasmid. However, plasmids are unstable and are often lost from the host cells if there is no selective pressure during the cultivation of the host cells. Another way to increase the copy number of the gene of interest is to integrate it into the host cell chromosome in multiple copies. It has previously been described how to integrate a gene into the chromosome by double homologous recombination without using antibiotic markers (Hone et al., Microbial Pathogenesis, 1988, 5: 407-418); integration of two genes has also been described (Novo Nordisk: WO 91/09129 and WO 94/14968). Integrating several copies of a gene into the chromosome of a host cell could lead to instability. Integration of two genes closely spaced in anti-parallel tandem to achieve better stability has been described (Novozymes: WO 99/41358)as well as the stable chromosomal multi-copy integration of genes (Novozymes: WO 02/00907).

Other ways of increasing the product yield would be to increase promoter activity of the specific promoter regulating the expression of a specific gene of interest. Also a more general increase in the activity of several promoters at the same time could lead to an improved product yield.

The most studied bacterial RNA-polymerase is the *E. coli* RNA-polymerase and it is representative of the enzymes isolated from a number of bacterial genera, including *Salmonella, Serratia, Proteus, Aerobacter,* and *Bacillus* (Fukuda et al., 1977, Mol. Gen. Genet. 154:135).

The RNA-polymerase core enzyme is composed of alpha, beta, and beta' subunits in the ratio 2:1:1, encoded for by the *rpoA, rpoB,* and *rpoC* genes respectively. Mutations that confer a cellular resistance to several antibiotics (e.g. rifampicin) are within the *rpoB* gene.

Rifampicin binds to the beta-subunit and completely blocks productive initiation of RNA chains by the enzyme in vitro and in vivo (Wehrli and Staehelin, 1971, Bact. Rev. 35:290). Cells gain resistance to rifampicin by virtue of an altered beta subunit that fails to bind the drug.

Mutations in the beta-subunit of the RNA-polymerase of rifampicin resistant cells map in three separate regions within a 200-amino-acid stretch in the center of the subunit, and these have been mentioned as a putative rifampicin-binding pocket (Jin and Gross, 1988, J. Mol. Biol. 202: 45-58).

Mutations in *rpoB* resulting in rifampicin resistance have been reported to cause highly pleiotropic phenotypes (Yang and Price, 1995, J. Biol. Chem. 270: 23930-23933; Kane et al., 1979, J. Bacteriol. 137: 1028-1030). In some cases resulting in an increase in gene activity and in other cases with no effect (Kane et al., 1979, J. Bacteriol. 137: 1028-1030). Sharipova et al.(1994, Microbiology 63: 29-32) have reported higher levels of phosphatase activity for some rifampicin resistant strains and lower levels for other rifampicin resistant strains. A 100-fold improved alpha-amylase activity has been reported in a rifampicin resistant *Spo⁻* isolate of *Bacillus licheniformis* (Hu Xuezhi et al., 1991, Acta microbiologica sinica 31: 268-273). In *Bacillus subtilis* the mutation Q469R results in rifampicin resistance and hypersensitivity to NusG (Ingham and Furnaux, 2000, Microbiology, 146: 3041-3049).

Mutations in the beta-subunit of the eubacterial RNA-polymerase (rpoB), which confer rifampicin resistance, have also been disclosed by Ingham C J et al (Mutations in the beta subunit of the Bacillus subtilis RNA polymerase that confer both rifampicin resistance and hypersensitivity to NusG, 2000, Microbiology 146(12): 3041-3049).

### SUMMARY OF THE INVENTION

Though some reports have indicated an increased productivity of some gene products in rifampicin resistant isolates the effects of mutations in *rpoB* have been reported to be highly pleiotropic and the observed increase in productivity have never been linked to specific mutations in *rpoB.* Surprisingly it has now been discovered that the specific mutations of the present invention alone or in combination will result in a much improved productivity of a product of interest when the said *rpoB*-mutation(s) are present in the host strain.

In a first aspect the present invention relates to an isolated mutant of an otherwise isogenic parent *Bacillus* bacterium comprising at least one mutation resulting in a substitution of at least one amino acid in the beta-subunit of the RNA-polymerase encoded by the *rpoB*-gene providing an improved yield or productivity of a product of interest when compared to the yield or productivity in the isogenic parent strain grown at identical conditions, wherein the substitution of at least one amino acid occurs at any of the positions 469, 478, 482, 485, or 487 in SEQ ID NO:2, or at the equivalent positions in any RNA-polymerase beta-subunit family member; with the proviso that the mutant is not a *Bacillus subtilis* wherein the substitution of the at least one amino acid consists of Q469K, Q469R, or H482Y.

In a second aspect the present invention relates to a process for producing at least one product of interest in a mutant *Bacillus* bacterium *comprising* cultivating a mutant as defined above in a suitable medium whereby the said product is produced.

In a third aspect the present invention relates to a use of ae mutant according to the invention for producing at least one product of interest comprising cultivating the mutant in a suitable medium whereby the said product is produced.

### BRIEF DESCRIPTION OF DRAWINGS

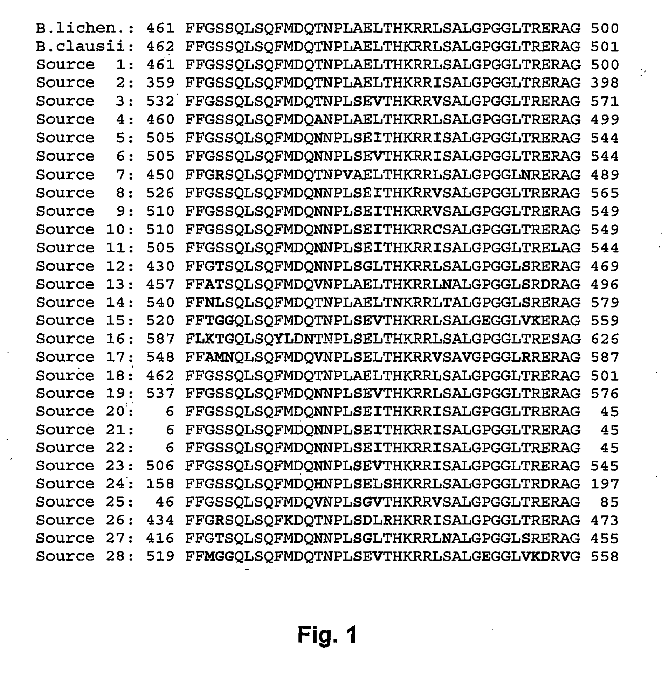

The top line of the aligned sequences in figure 1 shows the 40 amino acid segment of the *B. licheniformis* RpoB protein, from position 461 to 500 both incl. of SEO ID NO:2. The second line shows the homologous 40 amino acid segment of the *Bacillus clausii* RpoB protein, from position 462 to 501 both incl. of SEQ ID NO:4, aligned with the corresponding positions of SEQ ID NO:2. These two segments are aligned in figure 1 with published sequences of RpoB proteins from different bacteria, letters in bold indicate wildtype deviations from the RpoB sequence of the two *Bacillus* sequences at the top. The microbial sources of the homologous segments are indicated below for each source number in the figure:
Source 1) Boor et al., Genetic and transcriptional organization of the region encoding the beta subunit of Bacillus subtilis RNA polymerase. J. Biol. Chem. 270:20329 (1995).
Source 2) Kaneko et al., Sequence analysis of the genome of the unicellular cyanobacterium Synechocystis sp. strain PCC6803. II. Sequence determination of the entire genome and assignment of potential protein-coding regions. DNA Res. 3:109 (1996).
Source 3) Parkhill et al., Complete DNA sequence of a serogroup A strain of Neisseria menigitidis Z2491. Nature 404:502 (2000).
Source 4) Aboshkiwa et al., Cloning and physical mapping of the Staphylococcus aureus rplL, rpoB and rpoC genes, encoding ribosomal protein L7/L12 and RNA polymerase subunits beta and beta'. J. Gen. Microbiol. 138:1875 (1992).
Source 5) Ovchinnikov et al., The primary structure of Escherichia coli RNA polymerase. Nucleotide sequence of the rpoB gene and amino-acid sequence of the beta-subunit. Eur. J. Biochem. 116:621 (1981).
Source 6) Fleischmann et al., Whole-genome random sequencing and assembly of Haemophilus influenzae Rd. Science 269:496 (1995).
Source 7) Kalman et al., Comparative genomes of Chlamydia pneumoniae and C. trachomatis. Nat. Genet. 21:385 (1999).
Source 8) Mollet et al., Determination of Coxiella burnetii rpoB sequence and its use for phylogenetic analysis. Gene 207:97 (1998)
Source 9) Stover et al., Complete genome sequence of Pseudomonas aeruginosa PAO1, an opportunistic pathogen. Nature 406:959 (2000)
Source 10) Borodin et al., Nucleotide sequence of the rpoB gene coding for the beta-subunit of RNA polymerase in Pseudomonas putida. Dokl. Biochem. 302:1261 (1988)
Source 11) Sverdlov et al., Nucleotide sequence of the rpoB gene of Samonella typhimurium coding for the beta-subunit of RNA polymerase. Dokl. Biochem. 287:62 (1986)
Source 12) Honore et al., Nucleotide sequence of the first cosmid from the Mycobacterium leprae genome project: structure and function of the Rif-Str regions. Mol. Microbiol. 7:207 (1993).
Source 13) Alekshun et al., Molecular cloning and characterization of Borrelia burgdorferi rpoB. Gene 186:227 (1997).
Source 14) Laigret et al., The unique organization of the rpoB region of Spiroplasma citri: a restriction and modification system gene is adjacent to rpoB. Gene 171:95 (1996).
Source 15) Parkhill et al., The genome sequence of the food-borne pathogen Campylobacter jejuni reveals hypervariable sequences. Nature 403:665 (2000).
Source 16) Deckert et al., The complete genome of the hyperthermophilic bacterium Aquifex aeolicus. Nature 392:353 (1998)
Source 17) Palm et al., The DNA-dependent RNA-polymerase of Thermotoga maritima; characterisation of the enzyme and the DNA-sequence of the genes for the large subunits. Nucleic Acids Res. 21:4904 (1993).
Source 18) Takaki et al., Sequence analysis of a 32-kb region including the major ribosomal protein gene clusters from alkaliphilic Bacillus sp. strain C-125. Biosci. Biotechnol. Biochem. 63:452 (1999).
Source 19) Simpson et al., The genome sequence of the plant pathogen Xylella fastidiosa. Nature 406:151 (2000).
Source 20) Drancourt, M. Klebsiella ornithinolytica, Klebsiella taxonomy.Submitted (FEB-1999) to the EMBUGenBank/DDBJ databases.
Source 21) Drancourt and Raoult, Calymmatobacterium granulomatis rpoB.Submitted (DEC-1999) to the EMBUGenBank/DDBJ databases.
Source 22) Mollet et al., (Serratia marcescens) RNA polymerase beta-subunit.Submitted (NOV-1996) to the EMBUGenBank/DDBJ databases.
Source 23) Nakasone et al., Isolation of rpoB and rpoC genes from deep-sea piezophilic bacterium Shewanella violacea and its overexpression in Escherichia coli.Submitted (JUL-2000) to the EMBL/GenBank/DDBJ databases.
Source 24) Padayachee and Klugman, Molecular basis for rifampicin resistant Streptococcus pneumoniae isolates from South Africa.Submitted (FEB-1999) to the EMBL/GenBank/DDBJ databases.
Source 25) Nielsen et al., Legionella pneumophila RNA polymerase B-subunit (rpoB) gene.Submitted (DEC-1998) to the EMBUGenBank/DDBJ databases
Source 26) White et al., Genome sequence of the radioresistant bacterium Deinococcus radiodurans R1. Science 286:1571 (1999).
Source 27) Streptomyces coelicolor, Seeger and Harris, Submitted (MAR-2000) to the EMBUGenBank/DDBJ databases.
Source 28) Helicobacter pylori (Campylobacter pylori), Hocking et al., Submitted (JAN-1997) to the EMBUGenBank/DDBJ databases.

### DEFINITIONS

Prior to a discussion of the detailed embodiments of the invention, a definition of specific terms related to the main aspects of the invention is provided. In the present description and claims, the conventional one-letter codes for nucleotides and the conventional one-letter and three-letter codes for amino acid residues are used. For ease of reference the following nomenclature is used:
Original amino acid(s) position(s) substituted amino acid(s)
According to this nomenclature, and by way of example, the substitution of asparagine for alanine in position 30 is shown as:
Ala 30 Asn or A30N
a deletion of alanine in the same position is shown as:
Ala 30 * or A30*
and insertion of an additional amino acid residue, such as lysine, is shown as:
Ala 30 AlaLys or A30AK
A deletion of a consecutive stretch of amino acid residues, exemplified by amino acid residues 30-33, is indicated as (30-33)*.
Where a specific polypeptide contains a deletion (i.e. lacks an amino acid residue) in comparison with homologous polypeptides, and an insertion is made in such a position, this is indicated as: * 36 Asn or *36N
for insertion of an asparagine in position 36.

### Multiple mutations are separated by plus signs, i.e.:

Ala 30 Asn + Glu 34 Ser or A30N+E34S
representing substitutions in positions 30 and 34 (in which asparagine and serine is substituted for alanine and glutamic acid, respectively).

When one or more alternative amino acid residues may be inserted in a given position this is indicated as:
A30N,E or A30N or A30E

Furthermore, when a position suitable for modification is identified herein without any specific modification being suggested, it is to be understood that any other amino acid residue may be substituted for the amino acid residue present in that position (i.e. any amino acid residue - other than that normally present in the position in question - chosen among A, R, N, D, C, Q, E, G, H, I, L, K, M, F, P, S, T, W, Y and V). Thus, for instance, when a modification (replacement) of a methionine in position 202 is mentioned, M202, but not specified, it is to be understood that any of the other amino acids may be substituted for the methionine, i.e. any other amino acid chosen among A,R,N,D,C,Q,E,G,H,I,L,K,F,P,S,T,W,Y and V.

Mutant: The term "mutant "in the context of the present invention means the otherwise isogenic parent *Bacillus* bacterium which has obtained a mutation in at least one of the five claimed positions in the beta-subunit of the RNA-polymerase.

Substitution: The term "substitution" in the context of the present invention means the replacement of one amino acid with another amino acid.

Beta-subunit: The term "beta-subunit" in the context of the present invention means the RpoB-protein encoded for by the *rpoB* gene, and which subunit is comprised in the RNA-polymerase composed of alpha, beta, beta' subunits in the ratio 2:1:1 and encode for by the genes *rpoA, rpoB,* and *rpoC* respectively.

*rpoB*-gene: The term "*rpoB*-gene" in the context of the present invention means the gene encoding the beta-subunit of the RNA-polymerase.

Beta-subunit family member: The term " beta-subunit family member " in the context of the present invention does not mean family in the normal taxonomic sense where a family comprises members of the same genus, rather in the present context the term refers to any prokaryotic RNA-polymerase composed of three subunits alpha, beta, and beta' in the above mentioned ratio and wherein the amino acid sequence of said beta-subunit comprises a 40 amino acid contiguous sequence that can be aligned with the sequence of the RpoB protein from position 461 to 500 of SEQ ID NO:2 and result in at least 75% homology.

Isogenic parent strain: The term "isogenic parent strain" in the context of the present invention means a strain which is genetically identical to the progeny mutant or mutant strain of the present invention, except for the at least one mutation in the *rpoB*-gene of the said mutant.

Equivalent positions: The term "equivalent positions" in the context of the present invention means the positions after alignment with the segment from position 461 to 500 of SEQ ID NO:2 as also illustrated in Fig. 1 and in example 3.

Homology: The term "homology" in the context of the present invention relates to homologous polynucleotides or polypeptides. If two or more polynucleotides or two or more polypeptides are homologous, this means that the homologous polynucleotides or polypeptides have a "degree of identity" of at least 60%, more preferably at least 70%, even more preferably at least 85%, still more preferably at least 90%, more preferably at least 95%, and most preferably at least 98%. Whether two polynucleotide or polypeptide sequences have a sufficiently high degree of identity to be homologous as defined herein, can suitably be investigated by aligning the two sequences using a computer program known in the art, such as "GAP" provided in the GCG program package (Program Manual for the Wisconsin Package, Version 8, August 1994, Genetics Computer Group, 575 Science Drive, Madison, Wisconsin, USA 53711)(Needleman, S.B. and Wunsch, C.D., (1970), Journal of Molecular Biology, 48, 443-453). Using GAP with the following settings for DNA sequence comparison: GAP creation penalty of 5.0 and GAP extension penalty of 0.3."

Metabolic pathway: The term "metabolic pathway" in the context of the present invention means a chain of enzyme-catalyzed biochemical reactions in living cells which, e.g., convert one compound into another, or build up large macromolecules from smaller units, or break down compounds to release usable energy.

Exogenous: The term "exogenous" in the context of the present invention means that e.g. the gene is not normally present in the host organism in nature.

Endogenous: The term "endogenous" in the context of the present invention means that e.g. the gene originates from within the host organism.

Operon: The term "operon" in the context of the present invention means a polynucleotide comprising several genes that are clustered and perhaps even transcribed together into a polycistronic mRNA, e.g. genes coding for the enzymes of a metabolic pathway. The transcription of an operon may be initiated at a promoter region and controlled by a neighboring regulatory gene, which encodes a regulatory protein, which in turn binds to the operator sequence in the operon to respectively inhibit or enhance the transcription.

Altered product production: The term "altered product production" in the context of the present invention means that either the product yield is altered, which means the final amount of product produced per added amount of substrate is altered, or that the same amount of product is obtained by a shorter or longer culture period. An altered product production may be an increased product yield, or an increased productivity, however it may also be of interest to lower product yield, or to lower the productivity of certain products.

Nucleic acid construct: When used herein, the term "nucleic acid construct" means a nucleic acid molecule, either single- or double-stranded, which is isolated from a naturally occurring gene or which has been modified to contain segments of nucleic acids in a manner that would not otherwise exist in nature. The term nucleic acid construct is synonymous with the term "expression cassette" when the nucleic acid construct contains the control sequences required for expression of a coding sequence of the present invention.

Control sequence: The term "control sequences" is defined herein to include all components, which are necessary or advantageous for the expression of a polypeptide of the present invention. Each control sequence may be native or foreign to the nucleotide sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleotide sequence encoding a polypeptide.

Operably linked: The term "operably linked" is defined herein as a configuration in which a control sequence is appropriately placed at a position relative to the coding sequence of the DNA sequence such that the control sequence directs the expression of a polypeptide.

Coding sequence: When used herein the term "coding sequence" is intended to cover a nucleotide sequence, which directly specifies the amino acid sequence of its protein product. The boundaries of the coding sequence are generally determined by an open reading frame, which usually begins with the ATG start codon. The coding sequence typically include DNA, cDNA, and recombinant nucleotide sequences.

Expression: In the present context, the term "expression" includes any step involved in the production of the polypeptide including, but not limited to, transcription, post-transcriptional modification, translation, post-translational modification, and secretion.

Expression vector: In the present context, the term "expression vector" covers a DNA molecule, linear or circular, that comprises a segment encoding a polypeptide of the invention, and which is operably linked to additional segments that provide for its transcription.

Host cell: The term "host cell", as used herein, includes any cell type which is susceptible to transformation with a nucleic acid construct.

### DETAILED DESCRIPTION OF THE INVENTION

In *E. coli* mutations resulting in rifampicin resistance displays pleiotropic effects as discussed above. In *E. coli* this resistance is due to point mutations in the *rpoB* gene (Jin and Gross, 1988, J. Mol. Biol. 202: 45-58) mapping within three separate regions of the protein. In some reported cases the mutations also result in changed levels of specific proteins. Among the known *rpoB* genes from different bacteria that have been sequenced a high degree of homology have been found.

In *Bacillus licheniformis* we have determined the amino acid sequence of the RpoB protein to be about 95% homologous to the RpoB protein from *Bacillus subtilis.* Four colinear blocks of sequence similarity are shared between the *B. subtilis* and *E .coli* beta-subunits including conserved regions where alterations causing rifampicin resistance maps. In the present invention *rpoB*-mutants of *B. licheniformis* were isolated and tested for the production of a product of interest. RpoB-mutants can be obtained by known techniques in the art such as site directed mutagenesis or random mutagenesis. Also several positions in the RpoB-protein has previously been shown when mutated to result in rifampicin resistance.

In the present invention rifampicin resistant RpoB mutants of *B. licheniformis* and *B*. *clausii* were obtained, and mutants carrying different individual mutations in RpoB were tested for altered product production of a product of interest.

The analysis of the isolated mutants showed, that rifampicin resistance in *B*. *licheniformis* and *B. clausii* is due to point mutations in the *rpoB* gene, and furthermore specific mutations were identified, that consistently resulted in an increase in the production of specific products of interest as shown in examples 4 and 5.

In most cases a single substitution of one amino acid in one of 5 specific positions in the RpoB protein will result in an improved production of a product of interest, there may be synergistic effects of combining two or more of the specific mutations.

In a first aspect the present invention therefore relates to an isolated mutant of an otherwise isogenic parent *Bacillus* bacterium comprising at least one mutation resulting in a substitution of at least one amino acid in the beta-subunit of the RNA-polymerase encoded by the *rpoB*-gene providing an improved yield or productivity of a product of interest when compared to the yield or productivity in the isogenic parent strain grown at identical conditions, wherein the substitution of at least one amino acid occurs at any of the positions 469, 478, 482, 485, or 487 in SEQ ID NO:2, or at the equivalent positions in any RNA-polymerase beta-subunit family member; with the proviso that the mutant is not a *Bacillus subtilis* wherein the substitution of the at least one amino acid consists of Q469K, Q469R, or H482Y.

From the DNA sequence of the isolated mutants several specific mutations resulting in amino acid substitutions of at least one amino acid and providing an improved production of a product of interest have been identified. In one embodiment the present invention therefore relates to a substitution of at least one amino acid in the in the beta-subunit of the RNA-polymerase encoded for by the *rpoB*-gene as described above, wherein the said at least one substitution of at least one amino acid comprises Q469R, A478D, A478V, H482R, H482P, R485H, or S487L, wherein the positions correspond to the equivalent positions of those beta-subunits when aligned with SEQ ID NO:2.

Once the relevant positions resulting in an improved product production has been identified it will be obvious to the skilled person to try random substitutions at the same positions and in a further embodiment the present invention relates to an isolated mutant of the first aspect wherein the said substitution of at least one amino acid comprises any random substitution at the positions 469 or 478 in SEQ ID NO:2, or wherein the substitution of at least one amino acid comprises any substitution at the positions 469, 478, and/or 487 in SEQ ID NO:2, or at the equivalent position(s) in any RNA-polymerase beta-subunit family member.

In a particular embodiment the present invention relates to an isolated mutant as defined above, wherein the substitution of at least one amino acid comprises Q469R, A478D, and/or S487L; or preferably wherein the said at least one substitution of at least one amino acid comprises Q469R or A478D; wherein the positions correspond to the equivalent positions of those beta-subunits when aligned with SEQ ID NO:2.

The isolated bacterium according to the present invention should comprise a RNA-polymerase composed of three subunits alpha, beta, and beta' in the above mentioned ratio and wherein the amino acid sequence of said beta-subunit comprises a 40 amino acid contiguous sequence that can be aligned with the sequence of the RpoB protein from position 461 to 500 of SEQ ID NO:2 and result in at least 75% homology.

In a particular embodiment the said homology is at least 80%. In a further particular embodiment the homology is 85% and in a still further embodiment the homology is 90%.

In some bacterial genera, e.g. some gram-negative bacteria like Escherichia, Salmonella, Neiseria and Pseudomonas, the equivalent position to position 478 in *B*. *licheniformis* (SEQ ID NO:2) is normally serine (S) in stead of alanine (A), as evident from Fig. 1, in which the amino acid segment from position 461 to 500 in SEQ ID NO:2 from *B*. *licheniformis* has been aligned with published sequences of RpoB-proteins from various bacteria.

The amino acids, serine and alanine, are very similar in structure and it is therefore not surprising that it will be possible to replace alanine with serine or the other way around, without affecting the functionality of the RpoB-protein.

The claimed substitution of an amino acid at position 478 from e.g. A478D or A478V, or a random substitution of A478 should therefore in the context of the present invention also comprise S478D or S478V, or any random substitution of S478.

In one embodiment the isolated mutant is a gram positive bacterium. Useful gram positive bacteria include but are not limited to *Bacillus* sp., e.g. *Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis,* and *Bacillus thuringiensis.*

In another embodiment the said bacterium comprises *Bacillus lentus, Bacillus licheniformis, Bacillus amyloliquefaciens, Bacillus clausii, Bacillus stearothermophilus,* and *Bacillus subtilis.*

In a further embodiment of the present invention the said bacterium is a *Bacillus licheniformis.*

According to the present invention the product of interest comprises a gene product or a product of a metabolic pathway the production of which is improved as a result of the at least one substitution of at least one amino acid in the in the beta-subunit of the RNA-polymerase encoded for by the *rpoB-*gene*.*

One preferred embodiment of the invention relates to a mutant of the first aspect, wherein the product of interest is a product of a gene or a metabolic pathway; preferably the gene encoding the product of interest is comprised in an operon; even more preferably the gene is exogenous or endogenous to the mutant; still more preferably the gene is present in at least two copies or in at least ten copies or even in at least 100 copies.

The gene or the operon can be carried on a suitable plasmid that can be stably maintained, e.g. capable of stable autonomous replication in the host cell (the choice of plasmid will typically depend on the compatibility of the plasmid with the host cell into which the plasmid is to be introduced) or it can be carried on the chromosome of the host. The said gene may be endogenous to the host cell in which case the product of interest is a protein naturally produced by the host cell and in most cases the gene will be in it normal position on the chromosome. If the gene encoding the product of interest is an exogenous gene, the gene could either be carried on a suitable plasmid or it could be integrated on the host chromosome. In one embodiment of the invention the *Bacillus* baterium is recombinant. Also the product of interest may in another embodiment be a recombinant protein.

Integration of the gene encoding the product of interest may be achieved in several ways known to the skilled person. The gene may be present in one, two or more copies on the chromosome.

Integration of two genes has been described in WO 91/09129 and WO 94/14968 (Novo Nordisk) the content of which is hereby incorporated by reference. Integration of two genes closely spaced in anti-parallel tandem to achieve better stability has been described in WO 99/41358 (Novo Nordisk) the content of which is hereby incorporated by reference, as well as the stable chromosomal multi-copy integration of genes described in WO 02/00907 (Novozymes A/S) the content of which is incorporated herein by reference.

The presence of the gene encoding the product of interest in several copies may also further improve the production of the said product. If integrated on the chromosome of the host cell the gene may be present in one, two, or more copies per chromosome. If carried on a plasmid the gene may be present in several hundred copies per cell. In one embodiment of the present invention the said gene is present in at least two copies. In another embodiment the said gene is present in at least ten copies, and in a still further embodiment of the present invention the gene is present in at least 100 copies.

Selection of chromosomal integrant has for convenience resulted in the use of selectable markers such as antibiotic resistance markers. However it is desirable if possible to avoid the use of antibiotic marker genes. WO 01/90393 discloses a method for the integration of a gene in the chromosome of a host cell without leaving antibiotic resistance markers behind in the strain, the content of which is hereby incorporated by reference.

In a further embodiment the present invention thus relates to an isolated mutant as defined above, wherein the gene encoding the said product of interest is integrated on the mutant host chromosome without leaving any antibiotic resistance marker genes at the site of integration.

The present invention also relates to nucleic acid constructs comprising a nucleotide sequence encoding a product of interest, which may be operably linked to one or more control sequences that direct the expression of the coding sequence in a suitable host cell under conditions compatible with the control sequences.

A nucleotide sequence encoding a polypeptide of interest may be manipulated in a variety of ways to provide for expression of the polypeptide. Manipulation of the nucleotide sequence prior to its insertion into a vector may be desirable or necessary depending on the expression vector. The techniques for modifying nucleotide sequences utilizing recombinant DNA methods are well known in the art.

The control sequence may be an appropriate promoter sequence, a nucleotide sequence which is recognized by a host cell for expression of the nucleotide sequence. The promoter sequence contains transcriptional control sequences, which mediate the expression of the polypeptide. The promoter may be any nucleotide sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention, especially in a bacterial host cell, are the promoters obtained from the E. coli lac operon, Streptomyces coelicolor agarase gene (dagA), Bacillus subtilis levansucrase gene (sacB), Bacillus licheniformis alpha-amylase gene (amyL), Bacillus stearothermophilus maltogenic amylase gene (amyM), Bacillus amyloliquefaciens alpha-amylase gene (amyQ), Bacillus licheniformis penicillinase gene (penP), Bacillus subtilis xylA and xylB genes, and prokaryotic beta-lactamase gene (Villa-Kamaroff et al., 1978, Proceedings of the National Academy of Sciences USA 75: 3727-3731), as well as the tac promoter (DeBoer et al., 1983, Proceedings of the National Academy of Sciences USA 80: 21-25). Further promoters are described in "Useful proteins from recombinant bacteria" in Scientific American, 1980, 242: 74-94; and in Sambrook et al., 1989, supra.

The control sequence may also be a suitable transcription terminator sequence, a sequence recognized by a host cell to terminate transcription. The terminator sequence is operably linked to the 3' terminus of the nucleotide sequence encoding the polypeptide. Any terminator which is functional in the host cell of choice may be used in the present invention.

The control sequence may also be a suitable leader sequence, a nontranslated region of an mRNA which is important for translation by the host cell. The leader sequence is operably linked to the 5' terminus of the nucleotide sequence encoding the polypeptide. Any leader sequence that is functional in the host cell of choice may be used in the present invention.

The control sequence may also be a polyadenylation sequence, a sequence operably linked to the 3' terminus of the nucleotide sequence and which, when transcribed, is recognized by the host cell as a signal to add polyadenosine residues to transcribed mRNA. Any polyadenylation sequence which is functional in the host cell of choice may be used in the present invention.

The control sequence may also be a signal peptide coding region that codes for an amino acid sequence linked to the amino terminus of a polypeptide and directs the encoded polypeptide into the cell's secretory pathway. The 5' end of the coding sequence of the nucleotide sequence may inherently contain a signal peptide coding region naturally linked in translation reading frame with the segment of the coding region which encodes the secreted polypeptide. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the polypeptide. However, any signal peptide coding region which directs the expressed polypeptide into the secretory pathway of a host cell of choice may be used in the present invention.

Effective signal peptide coding regions for bacterial host cells are the signal peptide coding regions obtained from the genes for Bacillus NCIB 11837 maltogenic amylase, Bacillus stearothermophilus alpha-amylase, Bacillus licheniformis subtilisin, Bacillus licheniformis beta-lactamase, Bacillus stearothermophilus neutral proteases (nprT, nprS, nprM), and Bacillus subtilis prsA. Further signal peptides are described by Simonen and Palva, 1993, Microbiological Reviews 57: 109-137.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for Bacillus subtilis alkaline protease (aprE), Bacillus subtilis neutral protease (nprT), Saccharomyces cerevisiae alpha-factor, Rhizomucor miehei aspartic proteinase, and Myceliophthora thermophila laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences which allow the regulation of the expression of the polypeptide relative to the growth of the host cell. Examples of regulatory systems are those which cause the expression of the gene to be turned on or off in response to a chemical or physical stimulus, including the presence of a regulatory compound. Regulatory systems in prokaryotic systems include the lac, tac, and trp operator systems. In yeast, the ADH2 system or GAL1 system may be used. In eukaryotic systems, these include the dihydrofolate reductase gene which is amplified in the presence of methotrexate, and the metallothionein genes which are amplified with heavy metals. In these cases, the nucleotide sequence encoding the polypeptide would be operably linked with the regulatory sequence.

### Expression Vectors

The present invention also relates to recombinant expression vectors comprising the nucleic acid construct of the invention. The various nucleotide and control sequences described above may be joined together to produce a recombinant expression vector which may include one or more convenient restriction sites to allow for insertion or substitution of the nucleotide sequence encoding the polypeptide at such sites. Alternatively, the nucleotide sequence of the present invention may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (e.g., a plasmid or virus) which can be conveniently subjected to recombinant DNA procedures and can bring about the expression of the nucleotide sequence. The choice of the vector will typically depend on the compatibility of the vector with the host cell into which the vector is to be introduced. The vectors may be linear or closed circular plasmids.

The vector may be an autonomously replicating vector, i.e., a vector which exists as an extrachromosomal entity, the replication of which is independent of chromosomal replication, e.g., a plasmid, an extrachromosomal element, a minichromosome, or an artificial chromosome.

The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors of the present invention preferably contain one or more selectable markers which permit easy selection of transformed cells. A selectable marker is a gene the product of which provides for biocide or viral resistance, resistance to heavy metals, prototrophy to auxotrophs, and the like.

Examples of bacterial selectable markers are the dal genes from Bacillus subtilis or Bacillus licheniformis, or markers which confer antibiotic resistance such as ampicillin, kanamycin, chloramphenicol or tetracycline resistance.

The vectors of the present invention preferably contain an element(s) that permits stable integration of the vector into the host cell's genome or autonomous replication of the vector in the cell independent of the genome.

For integration into the host cell genome, the vector may rely on the nucleotide sequence encoding the polypeptide or any other element of the vector for stable integration of the vector into the genome by homologous or nonhomologous recombination. Alternatively, the vector may contain additional nucleotide sequences for directing integration by homologous recombination into the genome of the host cell. The additional nucleotide sequences enable the vector to be integrated into the host cell genome at a precise location(s) in the chromosome(s). To increase the likelihood of integration at a precise location, the integrational elements should preferably contain a sufficient number of nucleotides, such as 100 to 1,500 base pairs, preferably 400 to 1,500 base pairs, and most preferably 800 to 1,500 base pairs, which are highly homologous with the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. Examples of bacterial origins of replication are the origins of replication of plasmids pBR322, pUC19, pACYC177, and pACYC184 permitting replication in E. coli, and pUB110, pE194, pTA1060, and pAMβ1 permitting replication in Bacillus. The origin of replication may be one having a mutation which makes its functioning temperature-sensitive in the host cell (see, e.g., Ehrlich, 1978, Proceedings of the National Academy of Sciences USA 75: 1433).

More than one copy of a nucleotide sequence of the present invention may be inserted into the host cell to increase production of the gene product. An increase in the copy number of the nucleotide sequence can be obtained by integrating at least one additional copy of the sequence into the host cell genome or by including an amplifiable selectable marker gene with the nucleotide sequence where cells containing amplified copies of the selectable marker gene, and thereby additional copies of the nucleotide sequence, can be selected for by cultivating the cells in the presence of the appropriate selectable agent.

The procedures used to ligate the elements described above to construct the recombinant expression vectors of the present invention are well known to one skilled in the art (see, e.g., Sambrook et al., 1989, supra).

### Host Cells

The present invention also relates to recombinant a host cell comprising the nucleic acid construct of the invention, which are advantageously used in the recombinant production of the polypeptides. A vector comprising a nucleotide sequence of the present invention is introduced into a host cell so that the vector is maintained as a chromosomal integrant or as a self-replicating extra-chromosomal vector as described earlier. The host cell may be a unicellular microorganism, e.g., a prokaryote, or a non-unicellular microorganism, e.g., a eukaryote.

Useful unicellular cells are bacterial cells such as gram positive bacteria including, but not limited to, a Bacillus cell, e.g., Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis, and Bacillus thuringiensis; or a Streptomyces cell, e.g., Streptomyces lividans or Streptomyces murinus, or gram negative bacteria such as E. coli and Pseudomonas sp. In a preferred embodiment, the bacterial host cell is a Bacillus lentus, Bacillus licheniformis, Bacillus stearothermophilus, or Bacillus subtilis cell. In another preferred embodiment, the Bacillus cell is an alkalophilic Bacillus.

The introduction of a vector into a bacterial host cell may, for instance, be effected by protoplast transformation (see, e.g., Chang and Cohen, 1979, Molecular General Genetics 168: 111-115), using competent cells (see, e.g., Young and Spizizin, 1961, Journal of Bacteriology 81: 823-829, or Dubnau and Davidoff-Abelson, 1971, Journal of Molecular Biology 56: 209-221), electroporation (see, e.g., Shigekawa and Dower, 1988, Biotechniques 6: 742-751), or conjugation (see, e.g., Koehler and Thorne, 1987, Journal of Bacteriology 169: 5771-5278).

The product of interest is any gene product or product of a metabolic pathway which is industrially useful and which can be produced in a bacterial cell. In one embodiment the product of interest comprises polypeptides, vitamins, amino acids, antibiotics, carbohydrates, or surfactants.

A preferred embodiment relates to a mutant of the first aspect, wherein the product of interest comprises polypeptides, vitamins, amino acids, antibiotics, carbohydrates, or surfactants; preferably the product of interest is a polypeptide, preferably an enzyme; still more preferably enzyme is an enzyme of a class selected from the group of enzyme classes consisting of oxidoreductases (EC 1), transferases (EC 2), hydrolases (EC 3), lyases (EC 4), isomerases (EC 5), and ligases (EC 6).

In another embodiment the enzyme is an enzyme with an activity selected from the group of enzyme activities consisting of aminopeptidase, amylase, amyloglucosidase, mannanase, carbohydrase, carboxypeptidase, catalase, cellulase, chitinase, cutinase, cyclodextrin glycosyltransferase, deoxyribonuclease, esterase, galactosidase, beta-galactosidase, glucoamylase, glucose oxidase, glucosidase, haloperoxidase, hemicellulase, invertase, isomerase, laccase, ligase, lipase, lyase, mannosidase, oxidase, pectinase, peroxidase, phytase, phenoloxidase, polyphenoloxidase, protease, ribonuclease, transferase, transglutaminase, or xylanase. Preferably the enzyme is an amylase or a mannanase.

In yet another embodiment the product is a polypeptide comprising cellulose binding domains, starch binding domains, antibodies, antimicrobial peptides, hormones, or fusion polypeptides. It is preferred, that the product of interest is a polypeptide which comprises cellulose binding domains, starch binding domains, antibodies, antimicrobial peptides, hormones, or fusion polypeptides. It is also preferred, that the product of interest is a carbohydrate, preferably hyaluronic acid.

When present in the host cell the specific mutations of the present invention result in an altered production of a product of interest. The altered production could e.g. be an improved yield or an improved productivity as defined above.

The altered production according to the invention is in one embodiment at least an additional 5% to 1000% of the normal level in the isogenic parent strain grown at identical conditions. In another embodiment the altered production is between 5% to 500%, in a further embodiment between 5% to 250%, and in a still further embodiment between 5% to 100%, and in still another embodiment between 5% to 50%.

The host cells of the present invention are cultured in a suitable nutrient medium under conditions permitting the production of the desired polypeptide, after which the resulting polypeptide optionally is recovered from the cells, or the culture broth.

The medium used to culture the cells may be any conventional medium suitable for growing the host cells, such as minimal or complex media containing appropriate supplements. Suitable media are available from commercial suppliers or may be prepared according to published recipes (e.g. in catalogues of the American Type Culture Collection). The media are prepared using procedures known in the art (see, e.g., references for bacteria and yeast; Bennett, J.W. and LaSure, L., editors, More Gene Manipulations in Fungi, Academic Press, CA, 1991).

If the polypeptide is secreted into the nutrient medium, the polypeptide can be recovered directly from the medium. If the polypeptide is not secreted, it can be recovered from cell lysates. The polypeptide may be recovered from the culture medium by conventional procedures including separating the host cells from the medium by centrifugation or filtration, precipitating the proteinaceous components of the supernatant or filtrate by means of a salt, e.g. ammonium sulphate, purification by a variety of chromatographic procedures, e.g. ion exchange chromatography, gelfiltration chromatography, affinity chromatography, or the like, dependent on the type of polypeptide in question.

The polypeptides may be detected using methods known in the art that are specific for the polypeptides. These detection methods may include use of specific antibodies, formation of an enzyme product, or disappearance of an enzyme substrate. For example, an enzyme assay may be used to determine the activity of the polypeptide.

The polypeptides of the present invention may be purified by a variety of procedures known in the art including, but not limited to, chromatography (e.g., ion exchange, affinity, hydrophobic, chromatofocusing, and size exclusion), electrophoretic procedures (*e.g*., preparative isoelectric focusing (IEF), differential solubility (*e.g.,* ammonium sulfate precipitation), or extraction (see, *e.g.,* Protein Purification, J.-C. Janson and Lars Ryden, editors, VCH Publishers, New York, 1989).

In one embodiment of the present invention the product of interest is secreted into the nutrient medium. In another embodiment the product is associated with the cell membrane, and in a still further embodiment the product is intracellular.

A second aspect of the invention relates to a process for producing at least one product of interest in a mutant comprising cultivating a mutant as defined in any of the embodiments of the first aspect of the invention in a suitable medium whereby the said product is produced. Suitable media for the cultivation is described above as well as methods for the purification or isolation of the produced product which is an optional additional step to the process of the present invention.

In the production methods of the present invention, the cells are cultivated in a nutrient medium suitable for production of the polypeptide using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If the polypeptide is secreted into the nutrient medium, the polypeptide can be recovered directly from the medium. If the polypeptide is not secreted, it can be recovered from cell lysates.

The polypeptides may be detected using methods known in the art that are specific for the polypeptides. These detection methods may include use of specific antibodies, formation of an enzyme product, or disappearance of an enzyme substrate. For example, an enzyme assay may be used to determine the activity of the polypeptide as described herein.

The resulting polypeptide may be recovered by methods known in the art. For example, the polypeptide may be recovered from the nutrient medium by conventional procedures including, but not limited to, centrifugation, filtration, extraction, spray-drying, evaporation, or precipitation.

The polypeptides of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, *e.g.,* Protein Purification, J.-C. Janson and Lars Ryden, editors, VCH Publishers, New York, 1989).

A third aspect of the present invention relates to the use of a mutant as defined in any of the embodiments of the first aspect of the invention for producing at least one product of interest comprising cultivating the said mutant in a suitable medium whereby the said product is produced, and optionally isolating or purifying the produced product.

The present invention is further illustrated by the following examples, which, however, are not to be construed as limiting the scope of protection. The features disclosed in the foregoing description and in the following examples may, both separately and in any combination thereof, be material for realising the invention in diverse forms thereof.

### EXAMPLES

### Example 1

### The DNA sequence of the rpoB gene from Bacillus licheniformis

The DNA sequence of the *rpoB* gene from *Bacillus licheniformis* was determined from plasmid clones containing fragments of *B. licheniformis* chromosomal DNA by standard techniques. The plasmid library containing the *B. licheniformis* clones was obtained from deposit ATCC14580. The DNA sequence is shown in SEQ ID NO:1.

### Example 2

### The B. licheniformis RpoB protein

The *B. licheniformis* RpoB protein as translated from the open reading frame of the DNA sequence in SEQ ID NO:1 is shown in SEQ ID NO:2.

### Example 3

### DNA polymerase segment alignment

The 40 amino acid segment of the *B. licheniformis* RpoB protein from position 461 to 500 in SEQ ID NO:2 was used in a BLAST search against published sequences of RpoB proteins from different bacteria. The alignment is shown in figure 1 with the *B. licheniformis* RpoB sequence at the top.

### Example 4

### Yield/productivity improvements in specific rpoB mutants

### Materials and methods

Expression cassettes were integrated in one or more copies into the chromosome of the strains below, as described in WO 91/09129 and WO 99/41358.

### Strains:

- JA 677:: A *B. licheniformis* overproducing a variant of its endogenous alpha-amylase (BLA).
- JA 678:: JA 677 with a mutation in *rpoB* resulting in the amino acid change Q469R.
- JA 688:: A *B. licheniformis* overproducing a variant of the *B.stearothermophilus* alpha-amylase (BSG).
- JA 689:: JA 688 with a mutation in *rpoB* resulting in the amino acid change Q469R.
- JA 690:: JA 688 with a mutation in *rpoB* resulting in the amino acid change H482R.
- JA 684:: A *B. licheniformis* overproducing the *B.amyloliquefaciens* alpha-amylase (BAN).
- JA 687:: JA 684 with a mutation in *rpoB* resulting in the amino acid change A478D
- SJ 4671:: A *B. licheniformis* overproducing its endogenous alpha-amylase (BLA).
- SJ 4671 rif10:: SJ 4671 with a mutation in *rpoB* resulting in the amino acid change A478V.
- SJ 4490:: A *B. licheniformis* overproducing its endogenous alpha-amylase.
- JA 675:: SJ4490 with a mutation in *rpoB* resulting in the amino acid changes Q469R and R485H.
- SJ 6129:: A *B. licheniformis* overproducing a variant of a *Bacillus* alpha-amylase (disclosed in WO 00/60060); (correction - the actual internal ref.no. is SJ 6093).
- SJ 6129 rif10:: SJ 6129 (SJ 6093) with a mutation in *rpoB* resulting in the amino acid change S487L.

### Media:

LB agar: 10 g/I peptone from casein; 5 g/I yeast extract; 10 g/I Sodium Chloride; 12 g/I Bacto-agar adjusted to pH 6.8 - 7.2. LB agar with 50 KAN: LB-agar with 50 mg/l of Kanamycin. M-9 buffer (deionized water is used): Di-Sodiumhydrogenphosphate 2 H2O 8,8 g/l; Potassiumdihydrogenphosphate 3 g/I; Sodium Chloride 4 g/I; Magnesium sulphate 7 H2O 0,2 g/l.
Med-F shake flask media (the concentrations given are after final mixing with deionized water): Part A: Maltodextrin 11 g/l; Casitone 6,2 g/I; Bacto-peptone 0,5; Yeast Extract 0,5 g/I; Magnesium sulphate 7 H2O 0,5 g/l; Calcium chloride 0,1 g/I; Citric acid 50 mg/l; trace metals (MnSO₄ H₂O 2,5 mg/l; FeSO₄ 7H₂O 9,9 mg/l; CuSO₄ 5H₂O 1,0 mg/l; ZnCl₂ 1,0 mg/l); Pluronic 0,1 g/I; pH adjusted to 6,7; Part B: 5 g/l Potassiumdihydrogenphosphate pH adjusted to 6,7 with NaOH. After sterilization for 20 min at 121°C part A and B are mixed 1:1.

### Procedure for shake flask evaluation of strains:

First the strain was grown on agar slants 1 day at 37°C. (LB-agar for SJ4671; SJ4671rif10; SJ4490; JA675, LB with 50 KAN for JA 677; JA 678; JA 688; JA 689; JA 690; JA684; JA687). The agar was then washed with M-9 buffer, and the optical density at 650 nm (OD_{650 nm}) of the resulting cell suspension was measured.

Each shake flask is inoculated with the same amount of cells based on the OD₆₅₀ₙₘ measurement. An inoculum strength of OD x ml cell suspension =0,1 was used in this case. Each strain was inoculated in 3 shake flasks. The shake flasks were incubated at 37°C at 300 rpm, and samples were taken after 1, 2 and 3 days. The pH, OD₆₅₀ₙₘ, and α-amylase activity was measured and the relative activity was determined per strain, and in turn the relative activity was calculated to the parent strain for each *rpoB* mutant strain. Results are shown in Table 1. It is clear from these results that these mutations in *rpoB* have a significant effect on the productivity / yield of the enzyme.

**Table 1: Relative amylase activity of rpoB mutant strains (% of parent strain).**

| | Day 1 | Day 2 | Day 3 |
|---|---|---|---|
| BLA amylase variant JA677 vs JA678 (Q469R) | 142 | 161 | 168 |
| BSG amylase variant JA688 vs JA689 (Q469R) | 67 | 114 | 107 |
| BSG amylase variant JA688 vs JA690 (H482R) | 195 | 256 | 197 |
| BAN amylase JA684 vs JA687 (A478D) | 162 | 154 | 151 |
| BLA amylase SJ4671 vs SJ4671 rif 10 (A478V) | 121 | 114 | 112 |
| BLA SJ4490 vs JA675 (Q469R + R485H) | N/A | N/A | 135 |
| Variant amylase SJ 6093 vs SJ 6129 (S487L) | 149 | 122 | 114 |

### Example 5

### The DNA sequence of a part of the rpoB gene from Bacillus clausii

The DNA sequence of a part of the *rpoB* gene from *Bacillus clausii* was determined from plasmid clones containing fragments of *B. clausii* chromosomal DNA by standard techniques. The plasmid library containing the *B. clausii* clones was obtained from deposit NCIB 10309. The part of the DNA sequence is shown in SEQ ID NO:3.

### Example 6

### The B. clausii RpoB protein

The *B. clausii* RpoB protein as translated from the open reading frame of the DNA sequence in SEQ ID NO:3 is shown in SEQ ID NO:4.

### Example 7

### DNA polymerase segment alignment

The 40 amino acid segment of the *B. clausii* RpoB position 462 to 501 of SEQ ID NO:4, homologous and equivalent in positions to the RpoB from *B.licheniformis* shown in positions 461 to 500 in SEQ ID NO:2 was used in a BLAST search against published sequences of RpoB proteins from different bacteria. The alignment is shown in figure 1 with the *B. licheniformis* RpoB sequence at the top and *B.clausii* as the second from the top sequence.

### Example 8

### Yield/productivity improvements in specific rpoB mutants

### Materials and methods

### Expression was performed from the native expression system.

### Strains:

### NCIB 10309: A B. clausii (producing its endogenous protease, BCP).

PP143: A classical mutant of *B.clausii* NCIB 10309, which has no mutation in DNA-region encoding the 40 amino acid segment of the *B. clausii* RpoB shown in position 462 to 501 in SEQ ID NO:4 which is homologous to the RpoB of *B. licheniformis* shown in position 461 to 500 in SEQ ID NO:2.
NN49201: PP143 with a mutation in the *rpoB-*gene resulting in the substitution A479D in the encoded RpoB within the 40 amino acid segment shown in position 462 to 501 in SEQ ID NO:4. The substitution A479D in SEQ ID NO:4 corresponds to the equivalent substitution A478D in SEQ ID NO:2, as can clearly be seen from the two top-most lines of the alignment in figure 1.

### Media:

Agar: A sutiable agar to support good growth of B. clausii e.g. B3-agar: Peptone 6 g/l; Pepticase 4 g/l; Yeast extract 3 g/l; Meat extract 1.5 g/I; Glucose. 1H2O 1 g/l; Agar 20 g/I use of deionised water. pH adjustment to 7.35 with NaOH/HCl; Sterilised at 121ºC for 40 min. After cooling to 40-50ºC, 10% v/v of 1 M NaHCO3, pH 9, sterilised by filtration and 10% v/v of 10% w/v dried skim milk in deionised water, sterilised at 121ºC for 40 min, is added. M-9 buffer (deionized water is used): Di-Sodiumhydrogenphosphate 2 H2O 8.8 g/I; Potassiumdihydrogenphosphate 3 g/I; Sodium Chloride 4 g/I; Magnesium sulphate 7 H2O 0.2 g/l.
Med-F shake flask media (the concentrations given are after final mixing with deionized water): Part A: Maltodextrin 11 g/l; Casitone 6.2 g/I; Bacto-peptone 0.5; Yeast Extract 0.5 g/I; Magnesium sulphate 7 H2O 0.5 g/I; Calcium chloride 0.1 g/I; Citric acid 50 mg/l; trace metals (MnSO₄ H₂O 2.5 mg/l; FeSO₄ 7H₂O 9.9 mg/l; CuSO₄ 5H₂O 1.0 mg/l; ZnCl₂ 1.0 mg/l); Pluronic 0.1 g/I; pH adjusted to 6.7; Sterilization for 20 min at 121°C. Part B: 5 g/I Potassiumdihydrogenphosphate; 28.6 g/I Sodiumcarbonate; 8,4 g/l sodium-hydrogencarbonate pH adjusted to 9.0 with H₃PO₄. This solution is sterile-filtered and used right away. After sterilization part A and B are mixed.

### Procedure for shake flask evaluation of strains:

First the strain was grown on agar slants 1 day at 37°C. The agar was then washed with M-9 buffer, and the optical density at 650 nm (OD_{650 nm}) of the resulting cell suspension was measured.

Each shake flask is inoculated with the same amount of cells based on the OD₆₅₀ₙₘ measurement. An inoculum strength of OD x ml cell suspension =0.1 was used in this case. Each strain was inoculated in 2 shake flasks. The shake flasks were incubated at 37°C at 300 rpm, and samples were taken after 1, 2 and 3 days. The pH, OD₆₅₀ₙₘ, and protease activity was measured and the relative activity was determined per strain, and in turn the relative activity was calculated to the parent strain for the *rpoB* mutant strain. Results are shown in Table 2. It is clear from these results that the mutation in *rpoB* has a significant effect on the productivity / yield of the enzyme.

**Table 2: Relative protease activity of rpoB mutant strains (% of parent strain).**

| | Day 1 | Day 2 | Day 3 |
|---|---|---|---|
| BCP protease variant PP143 vs NN49201 (A479D) | 159 | 144 | 159 |

## Claims

1. An isolated mutant of an otherwise isogenic parent *Bacillus* bacterium comprising at least one mutation resulting in a substitution of at least one amino acid in the beta-subunit of the RNA-polymerase encoded by the *rpoB-*gene providing an improved yield or productivity of a product of interest when compared to the yield or productivity in the isogenic parent strain grown at identical conditions, wherein the substitution of at least one amino acid occurs at any of the positions 469, 478, 482, 485, or 487 in SEQ ID NO:2, or at the equivalent positions in any RNA-polymerase beta-subunit family member; with the proviso that the mutant is not a *Bacillus subtilis* wherein the substitution of the at least one amino acid consists of Q469K, Q469R, or H482Y.

2. The isolated mutant of claim 1, wherein the substitution of at least one amino acid comprises Q469R, A478D, A478V, H482R, H482P, R485H, or S487L.

3. The isolated mutant of claim 1, wherein the substitution of at least one amino acid comprises any random substitution at the positions 469 or 478 in SEQ ID NO:2, or at the equivalent position in any RNA-polymerase beta-subunit family member.

4. The isolated mutant of claim 1, wherein the substitution of at least one amino acid comprises any substitution at the positions 469, 478, and/or 487 in SEQ ID NO:2, or at the equivalent position(s) in any RNA-polymerase beta-subunit family member.

5. The isolated mutant according to any of the preceding claims, wherein the substitution of at least one amino acid comprises Q469R, A478D and/or S487L.

6. The isolated mutant according to any of the preceding claims, wherein the substitution of at least one amino acid comprises Q469R or A478D.

7. The isolated mutant of any of the preceding claims, wherein the RpoB protein is at least 80% homologous within the equivalent region of said protein to the amino acid sequence from position 461 to 500 in SEQ ID NO:2.

8. The isolated mutant of claim 7, wherein the RpoB protein is at least 90% homologous within the equivalent region of said protein to the amino acid sequence from position 461 to 500 in SEQ ID NO:2.

9. The isolated mutant according to any of the preceding claims, wherein said bacterium comprises *Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis,* and *Bacillus thuringiensis.*

10. The isolated mutant according to any of the preceding claims, wherein said bacterium comprises *Bacillus lentus, Bacillus licheniformis, Bacillus amyloliquefaciens, Bacillus clausii, Bacillus stearothermophilus,* and *Bacillus subtilis.*

11. The isolated mutant according to any of the preceding claims, wherein said bacterium is *Bacillus licheniformis.*

12. The isolated mutant according to any of the preceding claims, wherein the product of interest is a product of a gene or a metabolic pathway.

13. The isolated mutant according to claim 12, wherein the gene is comprised in an operon.

14. The isolated mutant according to claim 12 or 13, wherein the gene is exogenous or endogenous to the mutant.

15. The isolated mutant according to any of claims 12 - 14, wherein the gene is present in at least two copies.

16. The isolated mutant according to any of claims 12 - 15, wherein the gene is present in at least ten copies.

17. The isolated mutant according to any of claims 12 -16, wherein the gene is present in at least 100 copies.

18. The isolated mutant according to any of the preceeding claims, wherein the product of interest comprises polypeptides, vitamins, amino acids, antibiotics, carbohydrates, or surfactants.

19. The isolated mutant according to claim 18, wherein the product of interest is a polypeptide, preferably an enzyme.

20. The isolated mutant according to claim 19, wherein the enzyme is an enzyme of a class selected from the group of enzyme classes consisting of oxidoreductases (EC 1), transferases (EC 2), hydrolases (EC 3), lyases (EC 4), isomerases (EC 5), and ligases (EC 6).

21. The isolated mutant according to claim 19 or 20, wherein the enzyme is an enzyme with an activity selected from the group of enzyme activities consisting of aminopeptidase, amylase, amyloglucosidase, mannanase, carbohydrase, carboxypeptidase, catalase, cellulase, chitinase, cutinase, cyclodextrin glycosyltransferase, deoxyribonuclease, esterase, galactosidase, beta-galactosidase, glucoamylase, glucose oxidase, glucosidase, haloperoxidase, hemicellulase, invertase, isomerase, laccase, ligase, lipase, lyase, mannosidase, oxidase, pectinase, peroxidase, phytase, phenoloxidase, polyphenoloxidase, protease, ribonuclease, transferase, transglutaminase, or xylanase.

22. The isolated mutant according to claim 20, wherein the enzyme is an amylase or a mannanase.

23. The isolated mutant according to claim 18, wherein the product of interest is a polypeptide which comprises cellulose binding domains, starch binding domains, antibodies, antimicrobial peptides, hormones, or fusion polypeptides.

24. The isolated mutant according to claim 18, wherein the product of interest is a carbohydrate, preferably hyaluronic acid.

25. The isolated mutant according to any of the preceding claims, wherein the *Bacillus* bacterium is recombinant.

26. The isolated mutant according to claim 25, wherein the product of interest is a recombinant polypeptide.

27. The isolated mutant according claim 26, wherein the polypeptide is encoded by a gene which is integrated on the mutant host chromosome without leaving any antibiotic resistance marker genes at the site of integration.

28. The isolated mutant according to any of the preceding claims, wherein the altered production of the product of interest is at least an additional 5% to 1000% of the normal level in the isogenic parent strain grown at identical conditions.

29. The isolated mutant according to any of the preceding claims, wherein the altered production of a product of interest is at least an additional 5% to 500% of the normal level in the isogenic parent strain grown at identical conditions.

30. The isolated mutant according to any of the preceding claims, wherein the altered production of a product of interest is at least an additional 5% to 250% of the normal level in the isogenic parent strain grown at identical conditions.

31. The isolated mutant according to any of the preceding claims, wherein the altered production of a product of interest comprises an improved yield or an improved productivity.

32. The isolated mutant according to any of the preceding claims, wherein the product of interest is secreted, membrane associated, or intracellular.

33. A process for producing at least one product of interest in a mutant *Bacillus* bacterium comprising cultivating a mutant as defined in any of the claims 1 - 32 in a suitable medium whereby the said product is produced.

34. The process according to claim 33 further comprising isolating or purifying the product of interest.

35. Use of a mutant as defined in any of claims 1 - 32 for producing at least one product of interest comprising cultivating the mutant in a suitable medium whereby the said product is produced.

36. The use according to claim 35 further comprising isolating or purifying the product of interest.

## Patentansprüche

1. Isolierte Mutante eines ansonsten isogenen parentalen *Bacillus*-Bakteriums umfassend mindestens eine Mutation, die eine Substitution mindestens einer Aminosäure in der Beta-Untereinheit der RNA-Polymerase, die durch das *rpoB-*Gen kodiert wird, zum Ergebnis hat, was eine verbesserte Ausbeute oder Produktivität eines interessierenden Produkts zur Verfügung stellt, verglichen mit der Ausbeute oder der Produktivität im isogenen parentalen Stamm, der unter identischen Bedingungen wachsen gelassen wird, wobei die Subtitution von mindestens einer Aminosäure an einer beliebigen der Positionen 469, 478, 482, 485 oder 487 in SEQ ID NR. 2 oder an den entsprechenden Positionen in einem beliebigen Mitglied der Familie der RNA-Polymerase-Beta-Untereinheiten auftritt; mit der Maßgabe, dass die Mutante nicht ein *Bacillus subtilis* ist, wobei die Substitution der mindestens einen Aminosäure aus Q469K, Q469R oder H482Y besteht.

2. Isolierte Mutante nach Anspruch 1, wobei die Substitution mindestens einer Aminosäure Q469R, A478D, A478V, H482R, H482P, R485H oder S487L umfasst.

3. Isolierte Mutante nach Anspruch 1, wobei die Substitution von mindestens einer Aminosäure eine beliebige zufällige Substitution an den Positionen 469 oder 478 in SEQ ID NR. 2 oder an der entsprechenden Position in einem beliebigen Mitglied der Familie der RNA-Polymerase-Beta-Untereinheiten umfasst.

4. Isolierte Mutante nach Anspruch 1, wobei die Substitution von mindestens einer Aminosäure eine beliebige Substitution an den Positionen 469, 478 und/oder 487 in SEQ ID NR. 2 oder an der/den entsprechenden Position(en) in einem beliebigen Mitglied der Familie der RNA-Polymerase-Beta-Untereinheiten umfasst.

5. Isolierte Mutante nach einem beliebigen der vorangehenden Ansprüche, wobei die Substitution mindestens einer Aminosäure Q469R, A478D und/oder S487L umfasst.

6. Isolierte Mutante nach einem beliebigen der vorangehenden Ansprüche, wobei die Substitution mindestens einer Aminosäure Q469R oder A478D umfasst.

7. Isolierte Mutante nach einem beliebigen der vorangehenden Ansprüche, wobei das RpoB-Protein innerhalb der entsprechenden Region des Proteins mindestens 80 % homolog zur Aminosäuresequenz von Position 461 bis 500 in SEQ ID NR. 2 ist.

8. Isolierte Mutante nach Anspruch 7, wobei das RpoB-Protein innerhalb der entsprechenden Region des Proteins mindestens 90 % homolog zur Aminosäuresequenz von Position 461 bis 500 in SEQ ID NR. 2 ist.

9. Isolierte Mutante nach einem beliebigen der vorangehenden Ansprüche, wobei das Bakterium *Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis* und *Bacillus thuringiensis* umfasst.

10. Isolierte Mutante nach einem beliebigen der vorangehenden Ansprüche, wobei das Bakterium *Bacillus lentus, Bacillus licheniformis, Bacillus amyloliquefaciens, Bacillus clausii, Bacillus stearothermophilus* und *Bacillus subtilis* umfasst.

11. Isolierte Mutante nach einem beliebigen der vorangehenden Ansprüche, wobei das Bakterium *Bacillus licheniformis* ist.

12. Isolierte Mutante nach einem beliebigen der vorangehenden Ansprüche, wobei das interessierende Produkt das Produkt eines Gens oder eines Stoffwechselweges ist.

13. Isolierte Mutante nach Anspruch 12, wobei das Gen in einem Operon umfasst ist.

14. Isolierte Mutante nach Anspruch 12 oder 13, wobei das Gen exogen oder endogen bezüglich der Mutante ist.

15. Isolierte Mutante nach einem beliebigen der Ansprüche 12-14, wobei das Gen in mindestens zwei Kopien vorhanden ist.

16. Isolierte Mutante nach einem beliebigen der Ansprüche 12-15, wobei das Gen in mindestens zehn Kopien vorhanden ist.

17. Isolierte Mutante nach einem beliebigen der Ansprüche 12-16, wobei das Gen in mindestens 100 Kopien vorhanden ist.

18. Isolierte Mutante nach einem beliebigen der vorangehenden Ansprüche, wobei das interessierende Produkt Polypeptide, Vitamine, Aminosäuren, Antibiotika, Kohlenhydrate oder Surfactants umfasst.

19. Isolierte Mutante nach Anspruch 18, wobei das interessierende Produkt ein Polypeptid, bevorzugt ein Enyzm ist.

20. Isolierte Mutante nach Anspruch 19, wobei das Enzym ein Enzym einer Klasse ausgewählt aus der Gruppe von Enzymklassen bestehend aus Oxidoreduktasen (EC 1), Transferasen (EC 2), Hydrolasen (EC 3), Lyasen (EC 4), Isomerasen (EC 5) und Ligasen (EC 6) ist.

21. Isolierte Mutante nach Anspruch 19 oder 20, wobei das Enzym ein Enzym mit einer Aktivität ausgewählt aus der Gruppe von Enzymaktivitäten bestehend aus Aminopeptidase, Amylase, Amyloglukosidase, Mannanase, Carbohydrase, Carboxypeptidase, Katalase, Cellulase, Chitinase, Cutinase, Cyclodextrin-Glykosyltransferase, Desoxyribonuklease, Esterase, Galaktosidase, Beta-Galaktosidase, Glucoamylase, Glucoseoxidase, Glukosidase, Haloperoxidase, Hemicellulase, Invertase, Isomerase, Laccase, Ligase, Lipase, Lyase, Mannosidase, Oxidase, Pektinase, Peroxidase, Phytase, Phenoloxidase, Polyphenoloxidase, Protease, Ribonuklease, Transferase, Transglutaminase oder Xylanase ist.

22. Isolierte Mutante nach Anspruch 20, wobei das Enzym eine Amylase oder eine Mannanase ist.

23. Isolierte Mutante nach Anspruch 18, wobei das interessierende Produkt ein Polypeptid ist, das Cellulose-Bindedomänen, Stärke-Bindedomänen, Antikörper, antimikrobielle Peptide, Hormone, oder Fusionspolypeptide umfasst.

24. Isolierte Mutante nach Anspruch 18, wobei das interessierende Produkt ein Kohlenhydrat, bevorzugt Hyaluronsäure ist.

25. Isolierte Mutante nach einem beliebigen der vorangehenden Ansprüche, wobei das Bacillus-Bakterium rekombinant ist.

26. Isolierte Mutante nach Anspruch 25, wobei das interessierende Produkt ein rekombinantes Polypeptid ist.

27. Isolierte Mutante nach Anspruch 26, wobei das Polypeptid durch ein Gen kodiert wird, das in das mutierte Wirtschromosom integriert ist, ohne irgendwelche Antibiotikaresistenz-Markergene an der Integrationsstelle zurückzulassen.

28. Isolierte Mutante nach einem beliebigen der vorangehenden Ansprüche, wobei die veränderte Herstellung des interessierenden Produkts mindestens zusätzliche 5 bis 1000 % der normalen Menge im isogenen parentalen Stamm, der unter identischen Bedingungen wachsen gelassen wird, beträgt.

29. Isolierte Mutante nach einem beliebigen der vorangehenden Ansprüche, wobei die veränderte Herstellung des interessierenden Produkts mindestens zusätzliche 5 bis 500 % der normalen Menge im isogenen parentalen Stamm, der unter identischen Bedingungen wachsen gelassen wird, beträgt.

30. Isolierte Mutante nach einem beliebigen der vorangehenden Ansprüche, wobei die veränderte Herstellung des interessierenden Produkts mindestens zusätzliche 5 bis 250 % der normalen Menge im isogenen parentalen Stamm, der unter identischen Bedingungen wachsen gelassen wird, beträgt.

31. Isolierte Mutante nach einem beliebigen der vorangehenden Ansprüche, wobei die veränderte Herstellung des interessierenden Produkts eine verbesserte Ausbeute oder eine verbesserte Produktivität umfasst.

32. Isolierte Mutante nach einem beliebigen der vorangehenden Ansprüche, wobei das interessierende Produkt sekretiert, an die Membran assoziiert, oder intrazellulär wird.

33. Verfahren zum Herstellen von mindestens einem interessierenden Produkt in einem mutierten Bacillus-Bakterium, umfassend das Kultivieren einer Mutante wie in einem beliebigen der Ansprüche 1-32 definiert in einem geeigneten Medium, wobei das Produkt hergestellt wird.

34. Verfahren nach Anspruch 33, weiter umfassend das Isolieren oder Reinigen des interessierenden Produkts.

35. Verwendung einer Mutante wie in einem beliebigen der Ansprüche 1-32 definiert, zum Herstellen von mindestens einem interessierenden Produkt, umfassend das Kultivieren der Mutante in einem geeigneten Medium, wobei das Produkt hergestellt wird.

36. Verwendung nach Anspruch 35, weiterhin umfassend das Isolieren oder Reinigen des interessierenden Produkts.

## Revendications

1. Mutant isolé d'une bactérie Bacillus parente par ailleurs isogénique, comprenant au moins une mutation aboutissant à une substitution d'au moins un acide aminé dans la sous-unité bêta de l'ARN-polymérase codée par le gène *rpoB* donnant un rendement amélioré ou une productivité améliorée d'un produit d'intérêt par rapport au rendement ou à la productivité dans la souche parente isogénique, cultivée dans des conditions identiques, dans lequel la substitution d'au moins un acide aminé se produit à l'une quelconque des positions 469, 478, 482, 485 ou 487 dans la SEQ ID N°2, ou à des positions équivalentes dans un membre quelconque de la famille des sous-unités bêta de l'ARN polymérase ; à la condition que le mutant ne soit pas un *Bacillus subtilis* dans lequel la substitution d'au moins un acide aminé consiste en Q469K, Q469R ou H482Y.

2. Mutant isolé selon la revendication 1, dans lequel la substitution d'au moins un acide aminé comprend Q469R, A478D, A478V, H482R, H482P, R485H ou S487L.

3. Mutant isolé selon la revendication 1, dans lequel la substitution d'au moins un acide aminé comprend une quelconque substitution aléatoire aux positions 469 ou 478 dans la SEQ ID N°2, ou en la position équivalente dans un membre quelconque de la famille des sous-unités bêta de l'ARN polymérase.

4. Mutant isolé selon la revendication 1, dans lequel la substitution d'au moins un acide aminé comprend une quelconque substitution aux positions 469, 478 et/ou 487 dans la SEQ ID N°2, ou en la ou les positions équivalentes dans un membre quelconque de la famille des sous-unités bêta de l'ARN polymérase.

5. Mutant isolé selon l'une quelconque des revendications précédentes, dans lequel la substitution d'au moins un acide aminé comprend Q469R, A478D et/ou S487L.

6. Mutant isolé selon l'une quelconque des revendications précédentes, dans lequel la substitution d'au moins un acide aminé comprend Q469R ou A478D.

7. Mutant isolé selon l'une quelconque des revendications précédentes, dans lequel la protéine RpoB est au moins à 80 % homologue pour la région de ladite protéine équivalente à la séquence d'acides aminés de la position 461 à 500 dans la SEQ ID N°2.

8. Mutant isolé selon la revendication 7, dans lequel la protéine RpoB est au moins à 90 % homologue pour la région de ladite protéine équivalente à la séquence d'acides aminés de la position 461 à 500 dans la SEQ ID N°2.

9. Mutant isolé selon l'une quelconque des revendications précédentes, dans lequel ladite bactérie comprend *Bacillus alkalophilus, Bacillus amyloliquefaciens, Bacillus brevis, Bacillus circulans, Bacillus clausii, Bacillus coagulans, Bacillus lautus, Bacillus lentus, Bacillus licheniformis, Bacillus megaterium, Bacillus stearothermophilus, Bacillus subtilis* et *Bacillus thuringiensis.*

10. Mutant isolé selon l'une quelconque des revendications précédentes, dans lequel ladite bactérie comprend *Bacillus lentus, Bacillus licheniformis, Bacillus amyloliquefaciens, Bacillus clausii, Bacillus stearothermophilus* et *Bacillus subtilis.*

11. Mutant isolé selon l'une quelconque des revendications précédentes, dans lequel ladite bactérie est *Bacillus licheniformis.*

12. Mutant isolé selon l'une quelconque des revendications précédentes, dans lequel le produit d'intérêt est un produit d'un gène ou d'une voie métabolique.

13. Mutant isolé selon la revendication 12, dans lequel le gène est compris dans un opéron.

14. Mutant isolé selon la revendication 12 ou 13, dans lequel le gène est exogène ou endogène au mutant.

15. Mutant isolé selon l'une quelconque des revendications 12 à 14, dans lequel le gène est présent en au moins deux exemplaires.

16. Mutant isolé selon l'une quelconque des revendications 12 à 15, dans lequel le gène est présent en au moins dix exemplaires.

17. Mutant isolé selon l'une quelconque des revendications 12 à 16, dans lequel le gène est présent en au moins 100 exemplaires.

18. Mutant isolé selon l'une quelconque des revendications précédentes, dans lequel le produit d'intérêt comprend des polypeptides, des vitamines, des acides aminés, des antibiotiques, des hydrates de carbone ou des tensioactifs.

19. Mutant isolé selon la revendication 18, dans lequel le produit d'intérêt est un polypeptide, de préférence une enzyme.

20. Mutant isolé selon la revendication 19, dans lequel l'enzyme est une enzyme d'une catégorie sélectionnée parmi le groupe constitué des catégories d'enzyme composé des oxydoréductases (EC 1), transférases (EC 2), hydrolases (EC 3), lyases (EC 4), isomérases (EC 5) et ligases (EC 6).

21. Mutant isolé selon la revendication 19 ou 20, dans lequel l'enzyme est une enzyme ayant une activité choisie parmi le groupe des activités enzymatiques constitué de l'aminopeptidase, l'amylase, l'amyloglucosidase, la mannanase, la carbohydrase, la carboxypeptidase, la catalase, la cellulase, la quitinase, la cutinase, la cyclodextrine glycosyltransférase, la désoxyribonucléase, l'estérase, la galactosidase, la bêta-galactosidase, la glucoamylase, la glucose oxydase, la glucosidase, l'haloperoxydase, l'hémicellulase, l'invertase, l'isomérase, la laccase, la ligase, la lipase, la lyase, la mannosidase, l'oxydase, la pectinase, la peroxydase, la phytase, la phénoloxydase, la polyphénoloxydase, la protéase, la ribonucléase, la transférase, la transglutaminase ou la xylanase.

22. Mutant isolé selon la revendication 20, dans lequel l'enzyme est une amylase ou une mannanase.

23. Mutant isolé selon la revendication 18, dans lequel le produit d'intérêt est un polypeptide qui comprend des domaines de liaison à la cellulose, des domaines de liaison à l'amidon, des anticorps, des peptides antimicrobiens, des hormones ou des polypeptides de fusion.

24. Mutant isolé selon la revendication 18, dans lequel le produit d'intérêt est un hydrate de carbone, de préférence l'acide hyaluronique.

25. Mutant isolé selon l'une quelconque des revendications précédentes, dans lequel la bactérie Bacillus est recombinante.

26. Mutant isolé selon la revendication 25, dans lequel le produit d'intérêt est un polypeptide recombinant.

27. Mutant isolé selon la revendication 26, dans lequel le polypeptide est codé par un gène qui est intégré sur le chromosome de l'hôte mutant sans laisser de gènes marqueurs de résistance aux antibiotiques au site d'intégration.

28. Mutant isolé selon l'une quelconque des revendications précédentes, dans lequel la production altérée d'un produit d'intérêt est au moins 5 % à 1000 % de plus que le taux normal dans la souche parente isogénique cultivée dans des conditions identiques.

29. Mutant isolé selon l'une quelconque des revendications précédentes, dans lequel la production altérée d'un produit d'intérêt est au moins 5 % à 500 % de plus que le taux normal dans la souche parente isogénique cultivée dans des conditions identiques.

30. Mutant isolé selon l'une quelconque des revendications précédentes, dans lequel la production altérée d'un produit d'intérêt est au moins 5 % à 250 % de plus que le taux normal dans la souche parente isogénique cultivée dans des conditions identiques.

31. Mutant isolé selon l'une quelconque des revendications précédentes, dans lequel la production altérée d'un produit d'intérêt comprend un rendement amélioré ou une productivité améliorée.

32. Mutant isolé selon l'une quelconque des revendications précédentes, dans lequel le produit d'intérêt est sécrété, associé à une membrane ou intracellulaire.

33. Procédé de production d'au moins un produit d'intérêt dans une bactérie Bacillus mutante, comprenant la culture d'un mutant tel que défini dans l'une quelconque des revendications 1 à 32 dans un milieu adéquat, moyennant quoi ledit produit est obtenu.

34. Procédé selon la revendication 33, comprenant en outre l'isolation ou la purification du produit d'intérêt.

35. Utilisation d'un mutant selon l'une quelconque des revendications 1 à 32 pour produire au moins un produit d'intérêt, comprenant la culture du mutant dans un milieu approprié, moyennant quoi ledit produit est obtenu.

36. Utilisation selon la revendication 35, comprenant en outre l'isolation ou la purification du produit d'intérêt.


### SEQUENCE LISTING

<110> Novozymes A/S
   Jørgensen, Steen
   Andersen, Jens Tonne
   Banke, Niels
   Nielsen, Preben
<120> Eubacterial RNA-polymerase mutants with altered product production
<130> 10245.204-WO
<160> 4
<170> PatentIn version 3.2
<210> 1
   <211> 3579
   <212> DNA
   <213> Bacillus licheniformis
<220>
   <221> misc_feature
   <222> (1)..(3579)
   <223> DNA encoding the beta-subunit of the RNA-polymerase, RpoB, shown in SEQ ID NO:2.
<400> 1
   ttgacaggtc aactagttca gtatggacga caccgccagc gcagaagcta tgcacgcatt      60
   agcgaagtgt tagaattacc aaatctcatt gaaattcaaa cctcttctta tcagtggttt     120
   cttgatgagg gtcttagaga gatgtttcaa gacatatcgc caattgagga tttcactggt     180
   aacctctctc tggaatttat cgactacagc ttgggcgagc ctaagtatcc ggtagaagaa     240
   tcaaaagagc gggatgtgac ctattcagct ccgctgcggg ttaaagtccg cttaatcaac     300
   aaagaaaccg gcgaagtaaa ggatcaggat gtcttcatgg gcgatttccc tattatgaca     360
   gacactggaa ccttcattat caacggtgca gaacgggtca tcgtatctca gctcgttcgt     420
   tctccaagtg tatattttag tggtaaagta gacaaaaacg gtaagaaagg ttttaccgcg     480
   actgtcattc caaaccgtgg cgcatggtta gaatacgaga ctgatgcgaa agatgttgtt     540
   tacgtacgca tcgatcgcac acgtaagttg ccggttacgg ttcttttgcg tgctcttggc     600
   ttcggatctg accaagagat cattgatctc atcggtgaaa acgagtatct gcgcaatacg     660
   cttgataaag ataatacgga aaacaccgat aaagcgcttc ttgaaatcta cgagcgtctt     720
   cgtccagggg agccgcctac cgtagaaaac gcaaaaagcc tgcttgattc aaggttcttt     780
   gatccgaaaa gatatgacct tgcgagtgta ggacgttata aaattaataa aaagcttcac     840
   atcaaaaacc gactttttaa tcagcggctt gctgaaacgc tagtcgaccc tgaaacaggc     900
   gaaatccttg ccgaaaaagg agcgatttta gacagaagaa cgcttgataa agttctgccg     960
   taccttgaaa acggaatcgg ttttaaaaag ctttatccga acggcggagt tgtcgaagac    1020
   gaagtaacgc ttcagtctat caaaatctat gctccgacag accaagaagg ggagcagaca    1080
   atcaatgtga ttggaaatgc ttatatcgaa gaaggcgtta agaatattac accttctgat    1140
   attatcgctt ccatcagcta tttctttaac ctgcttcacg gagtgggcga taccgacgat 1200
   atcgaccatc taggaaaccg ccgtctccgt tcagtgggag agcttctgca aaaccaattc 1260
   cgtattggtt taagcagaat ggagcgcgtt gttcgtgaaa gaatgtctat tcaagataca 1320
   aacacgatca cgccgcagca gctgatcaat attcgccctg tcatcgcatc aatcaaagag 1380
   tttttcggaa gctcgcagct ttctcagttt atggatcaga cgaatccgct tgctgagctg 1440
   acgcataagc gccgtctgtc agcgctcgga ccgggcggtt tgactcgtga gcgcgccgga 1500
   atggaagtcc gtgacgttca ctattcacac tacggccgga tgtgtccgat tgaaacccct 1560
   gagggtccaa acatcggctt gatcaactcg ctttcttcat tcgcgaaagt gaaccgtttc 1620
   ggcttcatcg aaacgccgta tcgccgcgtc gatcctgaaa ctggaaaagt aacgccgaga 1680
   atcgattact tgacagctga tgaagaagac aactatgtcg ttgcccaggc aaacgcacgc 1740
   ctgaatgatg acggttcttt tgtggatgac agcatcgtcg cccgtttcag aggggagaac 1800
   accgttgttc cgaaagaccg cgtcgactat atggacgttt cgcctaaaca ggttgtctct 1860
   gccgcgactg catgtattcc tttcttggaa aacgatgact caaaccgcgc ccttatggga 1920
   gcgaacatgc aacgtcaggc cgtacctctt atgcagcctg aatcgccgat cgtcggaacc 1980
   gggatggaat atgtatctgc gaaagactcc ggtgccgctg ttatttgccg ccaccctgga 2040
   atcgttgaac gggtggaagc gaagaacatc tgggtgcgcc gctatgaaga agtcgacggc 2100
   cagaaagtca aagggaacct tgataaatac agcctgctga agtttgtccg ttccaaccag 2160
   ggaacttgct acaaccagcg tccgatcgta agcgtcggtg atgaggttga aaaaggtgaa 2220
   attttagctg acggtccgtc catggaaaaa ggtgagcttg cccttggacg caacgtcatg 2280
   gtcggcttta tgacatggga tggctacaac tatgaggatg ccatcatcat gagcgaacgc 2340
   cttgtaaaag acgacgtata cacgtctatt catattgaag aatacgaatc agaggcccgg 2400
   gatacaaaac tcggacctga agaaatcact cgcgatattc cgaacgtcgg tgaggacgct 2460
   cttcgcaatc tcgatgaacg cggaattatc cgtgtcggtg ctgaagtaaa agacggagat 2520
   cttcttgttg gtaaagtaac ccctaaaggt gttacagagc ttacggcgga agaacgcctg 2580
   cttcatgcca tcttcgggga aaaagcgcgt gaagtgcgtg atacgtcgct gcgtgtacct 2640
   cacggaggcg gcggtatcat ccttgatgta aaagtgttca accgcgaaga cggagacgaa 2700
   ctgcctccgg gcgttaacca gctcgtccgc gtctacatcg ttcagaagcg taaaatttct 2760
   gaaggggaca aaatggccgg acgccacggt aacaaaggtg ttatttcgaa aattcttccg 2820
   gaggaagata tgccgtatct gcctgacgga acgccgattg acatcatgtt aaacccgctg 2880
   ggcgtaccat cgcgtatgaa catcgggcag gtgttggagc tgcaccttgg tatggctgca 2940
   cgccgcctcg gtctgcatgt cgcgtcacct gtatttgacg gtgcccgcga agaagatgtg 3000
   tgggaaaccc ttgaagaagc cggcatgtca agagacgcaa aaaccgtcct ttacgacggc 3060
   cgaactgggg agccgtttga caaccgggtt tctgtcggca tcatgtacat gatcaaactg 3120
   gcacacatgg ttgacgacaa attgcacgca cgttcaaccg gtccttactc actcgttacc 3180
   cagcagcctc tcggaggtaa agcgcagttc ggcggacagc gttttggaga gatggaagtt   3240
   tgggcgcttg aagcttatgg tgcagcatat acgctacaag agatcctgac tgttaaatcg   3300
   gatgatgtcg taggccgtgt gaaaacatat gaagccatcg taaaaggcga caatgttcca   3360
   gaacctggtg ttccggaatc gttcaaagta ttgatcaaag agcttcaaag cttaggtatg   3420
   gacgtcaaaa ttctatcaag cgacgaagaa gaaatcgaaa tgagagactt ggaagacgac   3480
   gaagacgcga aacaaaacga agggctttct ctgccgaatg atgaagagtc cgaagagttg   3540
   gtttctgctg acgcagaacg cgatgtcgtt acaaaagaa                          3579
<210> 2
   <211> 1193
   <212> PRT
   <213> Bacillus licheniformis
<220>
   <221> PEPTIDE
   <222> (1)..(1193)
   <223> Beta-subunit of the RNA-polymerase, RpoB.
<400> 2
   Leu Thr Gly Gln Leu Val Gln Tyr Gly Arg His Arg Gln Arg Arg Ser
   1               5                   10                  15
   Tyr Ala Arg Ile Ser Glu Val Leu Glu Leu Pro Asn Leu Ile Glu Ile
               20                  25                  30
   Gln Thr Ser Ser Tyr Gln Trp Phe Leu Asp Glu Gly Leu Arg Glu Met
           35                  40                  45
   Phe Gln Asp Ile Ser Pro Ile Glu Asp Phe Thr Gly Asn Leu Ser Leu
       50                  55                  60
   Glu Phe Ile Asp Tyr Ser Leu Gly Glu Pro Lys Tyr Pro Val Glu Glu
   65                  70                  75                  80
   Ser Lys Glu Arg Asp Val Thr Tyr Ser Ala Pro Leu Arg Val Lys Val
                   85                  90                  95
   Arg Leu Ile Asn Lys Glu Thr Gly Glu Val Lys Asp Gln Asp Val Phe
               100                 105                 110
   Met Gly Asp Phe Pro Ile Met Thr Asp Thr Gly Thr Phe Ile Ile Asn
           115                 120                 125
   Gly Ala Glu Arg Val Ile Val Ser Gln Leu Val Arg Ser Pro Ser Val
       130                 135                 140
   Tyr Phe Ser Gly Lys Val Asp Lys Asn Gly Lys Lys Gly Phe Thr Ala
   145                 150                 155                 160
   Thr Val Ile Pro Asn Arg Gly Ala Trp Leu Glu Tyr Glu Thr Asp Ala
   165 170 175
   Lys Asp Val Val Tyr Val Arg Ile Asp Arg Thr Arg Lys Leu Pro Val
   180 185 190
   Thr Val Leu Leu Arg Ala Leu Gly Phe Gly Ser Asp Gln Glu Ile Ile
   195 200 205
   Asp Leu Ile Gly Glu Asn Glu Tyr Leu Arg Asn Thr Leu Asp Lys Asp
   210 215 220
   Asn Thr Glu Asn Thr Asp Lys Ala Leu Leu Glu Ile Tyr Glu Arg Leu
   225 230 235 240
   Arg Pro Gly Glu Pro Pro Thr Val Glu Asn Ala Lys Ser Leu Leu Asp
   245 250 255
   Ser Arg Phe Phe Asp Pro Lys Arg Tyr Asp Leu Ala Ser Val Gly Arg
   260 265 270
   Tyr Lys Ile Asn Lys Lys Leu His Ile Lys Asn Arg Leu Phe Asn Gln
   275 280 285
   Arg Leu Ala Glu Thr Leu Val Asp Pro Glu Thr Gly Glu Ile Leu Ala
   290 295 300
   Glu Lys Gly Ala Ile Leu Asp Arg Arg Thr Leu Asp Lys Val Leu Pro
   305 310 315 320
   Tyr Leu Glu Asn Gly Ile Gly Phe Lys Lys Leu Tyr Pro Asn Gly Gly
   325 330 335
   Val Val Glu Asp Glu Val Thr Leu Gln Ser Ile Lys Ile Tyr Ala Pro
   340 345 350
   Thr Asp Gln Glu Gly Glu Gln Thr Ile Asn Val Ile Gly Asn Ala Tyr
   355 360 365
   Ile Glu Glu Gly Val Lys Asn Ile Thr Pro Ser Asp Ile Ile Ala Ser
   370 375 380
   Ile Ser Tyr Phe Phe Asn Leu Leu His Gly Val Gly Asp Thr Asp Asp
   385 390 395 400
   Ile Asp His Leu Gly Asn Arg Arg Leu Arg Ser Val Gly Glu Leu Leu
   405 410 415
   Gln Asn Gln Phe Arg Ile Gly Leu Ser Arg Met Glu Arg Val Val Arg
   420 425 430
   Glu Arg Met Ser Ile Gln Asp Thr Asn Thr Ile Thr Pro Gln Gln Leu
   435 440 445
   Ile Asn Ile Arg Pro Val Ile Ala Ser Ile Lys Glu Phe Phe Gly Ser
   450 455 460
   Ser Gln Leu Ser Gln Phe Met Asp Gln Thr Asn Pro Leu Ala Glu Leu
   465 470 475 480
   Thr His Lys Arg Arg Leu Ser Ala Leu Gly Pro Gly Gly Leu Thr Arg
   485 490 495
   Glu Arg Ala Gly Met Glu Val Arg Asp Val His Tyr Ser His Tyr Gly
   500 505 510
   Arg Met Cys Pro Ile Glu Thr Pro Glu Gly Pro Asn Ile Gly Leu Ile
   515 520 525
   Asn Ser Leu Ser Ser Phe Ala Lys Val Asn Arg Phe Gly Phe Ile Glu
   530 535 540
   Thr Pro Tyr Arg Arg Val Asp Pro Glu Thr Gly Lys Val Thr Pro Arg
   545 550 555 560
   Ile Asp Tyr Leu Thr Ala Asp Glu Glu Asp Asn Tyr Val Val Ala Gln
   565 570 575
   Ala Asn Ala Arg Leu Asn Asp Asp Gly Ser Phe Val Asp Asp Ser Ile
   580 585 590
   Val Ala Arg Phe Arg Gly Glu Asn Thr Val Val Pro Lys Asp Arg Val
   595 600 605
   Asp Tyr Met Asp Val Ser Pro Lys Gln Val Val Ser Ala Ala Thr Ala
   610 615 620
   Cys Ile Pro Phe Leu Glu Asn Asp Asp Ser Asn Arg Ala Leu Met Gly
   625 630 635 640
   Ala Asn Met Gln Arg Gln Ala Val Pro Leu Met Gln Pro Glu Ser Pro
   645 650 655
   Ile Val Gly Thr Gly Met Glu Tyr Val Ser Ala Lys Asp Ser Gly Ala
   660 665 670
   Ala Val Ile Cys Arg His Pro Gly Ile Val Glu Arg Val Glu Ala Lys
   675 680 685
   Asn Ile Trp Val Arg Arg Tyr Glu Glu Val Asp Gly Gln Lys Val Lys
   690 695 700
   Gly Asn Leu Asp Lys Tyr Ser Leu Leu Lys Phe Val Arg Ser Asn Gln
   705 710 715 720
   Gly Thr Cys Tyr Asn Gln Arg Pro Ile Val Ser Val Gly Asp Glu Val
   725 730 735
   Glu Lys Gly Glu Ile Leu Ala Asp Gly Pro Ser Met Glu Lys Gly Glu
   740 745 750
   Leu Ala Leu Gly Arg Asn Val Met Val Gly Phe Met Thr Trp Asp Gly
   755 760 765
   Tyr Asn Tyr Glu Asp Ala Ile Ile Met Ser Glu Arg Leu Val Lys Asp
   770 775 780
   Asp Val Tyr Thr Ser Ile His Ile Glu Glu Tyr Glu Ser Glu Ala Arg
   785 790 795 800
   Asp Thr Lys Leu Gly Pro Glu Glu Ile Thr Arg Asp Ile Pro Asn Val
   805 810 815
   Gly Glu Asp Ala Leu Arg Asn Leu Asp Glu Arg Gly Ile Ile Arg Val
   820 825 830
   Gly Ala Glu Val Lys Asp Gly Asp Leu Leu Val Gly Lys Val Thr Pro
   835 840 845
   Lys Gly Val Thr Glu Leu Thr Ala Glu Glu Arg Leu Leu His Ala Ile
   850 855 860
   Phe Gly Glu Lys Ala Arg Glu Val Arg Asp Thr Ser Leu Arg Val Pro
   865 870 875 880
   His Gly Gly Gly Gly Ile Ile Leu Asp Val Lys Val Phe Asn Arg Glu
   885 890 895
   Asp Gly Asp Glu Leu Pro Pro Gly Val Asn Gln Leu Val Arg Val Tyr
   900 905 910
   Ile Val Gln Lys Arg Lys Ile Ser Glu Gly Asp Lys Met Ala Gly Arg
   915 920 925
   His Gly Asn Lys Gly Val Ile Ser Lys Ile Leu Pro Glu Glu Asp Met
   930 935 940
   Pro Tyr Leu Pro Asp Gly Thr Pro Ile Asp Ile Met Leu Asn Pro Leu
   945 950 955 960
   Gly Val Pro Ser Arg Met Asn Ile Gly Gln Val Leu Glu Leu His Leu
   965 970 975
   Gly Met Ala Ala Arg Arg Leu Gly Leu His Val Ala Ser Pro Val Phe
               980                 985                 990
   Asp Gly Ala Arg Glu Glu Asp Val Trp Glu Thr Leu Glu Glu Ala Gly
           995                 1000                1005
   Met Ser Arg Asp Ala Lys Thr Val Leu Tyr Asp Gly Arg Thr Gly
       1010                1015                1020
   Glu Pro Phe Asp Asn Arg Val Ser Val Gly Ile Met Tyr Met Ile
       1025                1030                1035
   Lys Leu Ala His Met Val Asp Asp Lys Leu His Ala Arg Ser Thr
       1040                1045                1050
   Gly Pro Tyr Ser Leu Val Thr Gln Gln Pro Leu Gly Gly Lys Ala
       1055                1060                1065
   Gln Phe Gly Gly Gln Arg Phe Gly Glu Met Glu Val Trp Ala Leu
       1070                1075                1080
   Glu Ala Tyr Gly Ala Ala Tyr Thr Leu Gln Glu Ile Leu Thr Val
       1085                1090                1095
   Lys Ser Asp Asp Val Val Gly Arg Val Lys Thr Tyr Glu Ala Ile
       1100                1105                1110
   Val Lys Gly Asp Asn Val Pro Glu Pro Gly Val Pro Glu Ser Phe
       1115                1120                1125
   Lys Val Leu Ile Lys Glu Leu Gln Ser Leu Gly Met Asp Val Lys
       1130                1135                1140
   Ile Leu Ser Ser Asp Glu Glu Glu Ile Glu Met Arg Asp Leu Glu
       1145                1150                1155
   Asp Asp Glu Asp Ala Lys Gln Asn Glu Gly Leu Ser Leu Pro Asn
       1160                1165                1170
   Asp Glu Glu Ser Glu Glu Leu Val Ser Ala Asp Ala Glu Arg Asp
       1175                1180                1185
   Val Val Thr Lys Glu
       1190
<210> 3
   <211> 3546
   <212> DNA
   <213> Bacillus clausii
<220>
   <221> misc_feature
   <222> (1)..(3546)
   <223> DNA encoding the beta-subunit of the RNA-polymerase, RpoB, shown in SEQ ID NO:4.
<400> 3
   ttgacaggtc aactaattca gtatggacgt caccgccagc ggagaagcta tgcgcgaatt      60
   aatgaagtgc tcgaactgcc aaacttgatt gaaattcaaa cagcttctta tcaatggttt     120
   cttgatgagg gtttgcggga gatgtttcaa gacatttcac cgatccaaga cttcactggc     180
   aatttagtgt tagagtttat tgattacagt ctaggagaac caaagtatcc tgttgatgag     240
   tcaaaagagc gtgacgtgac gtttgccgct cctctacgtg tcaaagttcg ccttattaat     300
   aaagagacag gcgaagtaaa agaacaggaa gtctttatgg gcgatttccc gttgatgaca     360
   gaaacaggga catttatcat caatggcgct gagcgcgtta tcgtttctca gcttgttcts     420
   tcaccgagcg tttactatag ccaaaagctc gacaaaaacg gaaaaaaagg ctttactgcc     480
   actgtcattc cgaaccgcgg agcgtggctt gagcttgaga cagatgcaaa agatattgtt     540
   tatgtacgta ttgaccgtac tcgtaaaatt ccagtaacag tacttttgcg tgctctaggc     600
   tttgggtctg atcaagaaat cgttgacctt ttaggcgaaa acgagtattt gcgcaacacg     660
   cttgagaaag ataatacgga ctcaactgat aaagtgctgc ttgaaatcta tgagcgtttg     720
   cgcccgggcg agccgccaac agtagaaaat gcgaaaagct tgcttgaatc tcgctttttc     780
   gatcctaagc gttatgacct cgcaaatgtc ggtcgttata aaataaataa aaagcttcat     840
   atcaaaaatc ggctatttaa ccaacgtttg gcggaaaagc ttgttgatcc ggaaacaggc     900
   gaagttctag ctgaagaggg aacacttctt gaccgcagaa cgttggataa actgatcccg     960
   catttggaga aaaatgttgg tttccgtaca gcccgtacat ctggcggcgt tcttgaggaa    1020
   tccgacgtcg aaatccaatc ggttaaaatt tatgtagcag atgactacga gggcgagcgc    1080
   gtcatttccg ttattagcaa cggcatggtt gaacgtgacg tgaagcatat tgcccctgct    1140
   gatatcatcg cttccatcag ctatttcttc aacttgctgc atggtgtcgg cgatacagac    1200
   gacattgacc atttgggcaa ccgccgtctc cgttcggttg gggaactgtt gcaaaaccaa    1260
   ttccgaattg gcctttctcg gatggaacgc gtcgtccgtg aacggatgtc gattcaagac    1320
   ccgaatgtaa ttacgccaca agcgcttatt aacattcgcc cagtcatcgc atcgataaaa    1380
   gagttctttg gcagctcgca gctttcacag ttcatggacc aaacgaaccc gctcgctgaa    1440
   ctgacgcata agcgccgtct ttccgcgctt ggcccaggcg gcttaactcg tgagcgcgct    1500
   ggaatggaag tgcgtgacgt tcactactcc cattacggcc ggatgtgtcc gattgaaacg    1560
   ccggaaggtc cgaacattgg cttaattaac acgctgtcct cttatgcgaa agtgaatgag    1620
   ttcggcttta tggaaacacc atatcgccgt gttgaccctg aaacagggaa agtgacagcg    1680
   cgtatcgact atttaacagc cgatgaagag gacaattatg tagttgccca agcaaatgcg    1740
   aagctgaatg aagacggatc gtttgtcgat gataacatca ttgcccgctt ccgcggtgaa    1800
   aacaccgtcg ttccttgcga ccgtgtcgac tatatggacg tttcgcctaa acaagttgtc    1860
   tctgcggcga cgtcatgtat tccgtttttg gaaaatgacg actcgaaccg cgcactaatg    1920
   ggcgcaaaca tgcagcgcca ggccgtgcct ttgctcgttc cagaagcacc gcttgtcgga 1980
   acagggatgg agcacgtgtc tgctaaagat tcaggggctg ctgttgtctc aaaatatgcc 2040
   ggtatcgttg aacgcgttac tgctaaagaa atttgggtcc gccgcattga agaagtagat 2100
   ggcaaagaaa cgaaaggcga ccttgataaa tacaaactac aaaaatttgt gcgctccaac 2160
   cagggcacaa gttataatca gcgcccgatt gtacgcgaag gcgatcgcgt tgaaaaacgt 2220
   gaaatcctcg cagatggtcc ttcaatggag atgggcgaaa tggcgcttgg ccgcaacgtg 2280
   cttgtcgcct ttatgacatg ggacggctac aactacgaag atgcgatcat tttaagtgag 2340
   cgcctagtca aagatgacgt ttatacgtcc atccatattg aggaatatga atcggatgcc 2400
   cgtgatacaa aactcggacc tgaagagatt acgcgcgata ttccgaacgt tggtaagaat 2460
   gcattacgca accttgatga gcggggaatt attcgcatcg gggctgaagt caaagatggc 2520
   gacattcttg ttggtaaagt gacgccaaag ggcgtaacgg agctgacggc ggaagaacgc 2580
   ctcttgcatg cgatttttgg agagaaagcc cgtgaagtgc gggatacttc attgcgtgca 2640
   ccgcatggtg gagacggaat cgttcttgac gtgaaaatct ttaaccgtga agacggcgat 2700
   gaacttcctc caggcgtcaa ccaacttgtc cgtgtctaca ttgtacaaaa gcggaaaatt 2760
   aaccagggcg ataaaatggc tggccgtcac gggaacaaag gtgttatttc ccgaatcctg 2820
   ccagaagaag atatgccgtt tttaccagac ggaacgcctg ttgacattat gttaaaccca 2880
   cttggcgtgc cttcgcggat gaatatcgga caagtgcttg aactccatct tggtatggct 2940
   gcccgcaagc taggcatcca tgttgcgtct ccagtatttg acggcgccag tgaagaagat 3000
   gtttggggca cattggaaga agcaggcatg gcccgagacg gaaaaacaat tttatatgat 3060
   ggccgtactg gcgaaccgtt tgacaaccgc gtatcagttg ggattatgta tatgatcaag 3120
   cttgcccata tggttgaacg accaagctcc atgcgcgtca acaggcccgt aactcattcg 3180
   ttaacgcagc agcttcttgg cggtaaagcc caatttggtg gacagcgttt cggggagatg 3240
   gaagtatggg cactggaagc atatggtgct gcttacacgc ttccaagaaa tccttcactg 3300
   ttaaattcag atgacgttgt tgggcgtgtg aaaacgtacg aggcaattgt aaaaggggaa 3360
   aacgttcctg agccaggggt gccagaatcg tttaaagtcc tcattaaaga attgcaatcg 3420
   ttgggcatgg atgtgaagat gctctccagc aacgaagagg aaattgaaat gcgtgagctg 3480
   gatgacgaag aagaccaaac gtctgaaaaa ctcaacctca acttagagac gaacgaatca 3540
   caacta 3546
<210> 4
   <211> 1182
   <212> PRT
   <213> Bacillus clausii
<220>
   <221> PEPTIDE
   <222> (1)..(1182)
   <223> Beta-subunit of the RNA-polymerase, RpoB.
<400> 4
   Leu Thr Gly Gln Leu Ile Gln Tyr Gly Arg His Arg Gln Arg Arg Ser
   1 5 10 15
   Tyr Ala Arg Ile Asn Glu Val Leu Glu Leu Pro Asn Leu Ile Glu Ile
   20 25 30
   Gln Thr Ala Ser Tyr Gln Trp Phe Leu Asp Glu Gly Leu Arg Glu Met
   35 40 45
   Phe Gln Asp Ile Ser Pro Ile Gln Asp Phe Thr Gly Asn Leu Val Leu
   50 55 60
   Glu Phe Ile Asp Tyr Ser Leu Gly Glu Pro Lys Tyr Pro Val Asp Glu
   65 70 75 80
   Ser Lys Glu Arg Asp Val Thr Phe Ala Ala Pro Leu Arg Val Lys Val
   85 90 95
   Arg Leu Ile Asn Lys Glu Thr Gly Glu Val Lys Glu Gln Glu Val Phe
   100 105 110
   Met Gly Asp Phe Pro Leu Met Thr Glu Thr Gly Thr Phe Ile Ile Asn
   115 120 125
   Gly Ala Glu Arg Val Ile Val Ser Gln Leu Val Leu Ser Pro Ser Val
   130 135 140
   Tyr Tyr Ser Gln Lys Leu Asp Lys Asn Gly Lys Lys Gly Phe Thr Ala
   145 150 155 160
   Thr Val Ile Pro Asn Arg Gly Ala Trp Leu Glu Leu Glu Thr Asp Ala
   165 170 175
   Lys Asp Ile Val Tyr Val Arg Ile Asp Arg Thr Arg Lys Ile Pro Val
   180 185 190
   Thr Val Leu Leu Arg Ala Leu Gly Phe Gly Ser Asp Gln Glu Ile Val
   195 200 205
   Asp Leu Leu Gly Glu Asn Glu Tyr Leu Arg Asn Thr Leu Glu Lys Asp
   210 215 220
   Asn Thr Asp Ser Thr Asp Lys Val Leu Leu Glu Ile Tyr Glu Arg Leu
   225 230 235 240
   Arg Pro Gly Glu Pro Pro Thr Val Glu Asn Ala Lys Ser Leu Leu Glu
   245 250 255
   Ser Arg Phe Phe Asp Pro Lys Arg Tyr Asp Leu Ala Asn Val Gly Arg
   260 265 270
   Tyr Lys Ile Asn Lys Lys Leu His Ile Lys Asn Arg Leu Phe Asn Gln
   275 280 285
   Arg Leu Ala Glu Lys Leu Val Asp Pro Glu Thr Gly Glu Val Leu Ala
   290 295 300
   Glu Glu Gly Thr Leu Leu Asp Arg Arg Thr Leu Asp Lys Leu Ile Pro
   305 310 315 320
   His Leu Glu Lys Asn Val Gly Phe Arg Thr Ala Arg Thr Ser Gly Gly
   325 330 335
   Val Leu Glu Glu Ser Asp Val Glu Ile Gln Ser Val Lys Ile Tyr Val
   340 345 350
   Ala Asp Asp Tyr Glu Gly Glu Arg Val Ile Ser Val Ile Ser Asn Gly
   355 360 365
   Met Val Glu Arg Asp Val Lys His Ile Ala Pro Ala Asp Ile Ile Ala
   370 375 380
   Ser Ile Ser Tyr Phe Phe Asn Leu Leu His Gly Val Gly Asp Thr Asp
   385 390 395 400
   Asp Ile Asp His Leu Gly Asn Arg Arg Leu Arg Ser Val Gly Glu Leu
   405 410 415
   Leu Gln Asn Gln Phe Arg Ile Gly Leu Ser Arg Met Glu Arg Val Val
   420 425 430
   Arg Glu Arg Met Ser Ile Gln Asp Pro Asn Val Ile Thr Pro Gln Ala
   435 440 445
   Leu Ile Asn Ile Arg Pro Val Ile Ala Ser Ile Lys Glu Phe Phe Gly
   450 455 460
   Ser Ser Gln Leu Ser Gln Phe Met Asp Gln Thr Asn Pro Leu Ala Glu
   465 470 475 480
   Leu Thr His Lys Arg Arg Leu Ser Ala Leu Gly Pro Gly Gly Leu Thr
   485 490 495
   Arg Glu Arg Ala Gly Met Glu Val Arg Asp Val His Tyr Ser His Tyr
   500 505 510
   Gly Arg Met Cys Pro Ile Glu Thr Pro Glu Gly Pro Asn Ile Gly Leu
   515 520 525
   Ile Asn Thr Leu Ser Ser Tyr Ala Lys Val Asn Glu Phe Gly Phe Met
   530 535 540
   Glu Thr Pro Tyr Arg Arg Val Asp Pro Glu Thr Gly Lys Val Thr Ala
   545 550 555 560
   Arg Ile Asp Tyr Leu Thr Ala Asp Glu Glu Asp Asn Tyr Val Val Ala
   565 570 575
   Gln Ala Asn Ala Lys Leu Asn Glu Asp Gly Ser Phe Val Asp Asp Asn
   580 585 590
   Ile Ile Ala Arg Phe Arg Gly Glu Asn Thr Val Val Pro Cys Asp Arg
   595 600 605
   Val Asp Tyr Met Asp Val Ser Pro Lys Gln Val Val Ser Ala Ala Thr
   610 615 620
   Ser Cys Ile Pro Phe Leu Glu Asn Asp Asp Ser Asn Arg Ala Leu Met
   625 630 635 640
   Gly Ala Asn Met Gln Arg Gln Ala Val Pro Leu Leu Val Pro Glu Ala
   645 650 655
   Pro Leu Val Gly Thr Gly Met Glu His Val Ser Ala Lys Asp Ser Gly
   660 665 670
   Ala Ala Val Val Ser Lys Tyr Ala Gly Ile Val Glu Arg Val Thr Ala
   675 680 685
   Lys Glu Ile Trp Val Arg Arg Ile Glu Glu Val Asp Gly Lys Glu Thr
   690 695 700
   Lys Gly Asp Leu Asp Lys Tyr Lys Leu Gln Lys Phe Val Arg Ser Asn
   705 710 715 720
   Gln Gly Thr Ser Tyr Asn Gln Arg Pro Ile Val Arg Glu Gly Asp Arg
   725 730 735
   Val Glu Lys Arg Glu Ile Leu Ala Asp Gly Pro Ser Met Glu Met Gly
   740 745 750
   Glu Met Ala Leu Gly Arg Asn Val Leu Val Ala Phe Met Thr Trp Asp
   755 760 765
   Gly Tyr Asn Tyr Glu Asp Ala Ile Ile Leu Ser Glu Arg Leu Val Lys
   770 775 780
   Asp Asp Val Tyr Thr Ser Ile His Ile Glu Glu Tyr Glu Ser Asp Ala
   785 790 795 800
   Arg Asp Thr Lys Leu Gly Pro Glu Glu Ile Thr Arg Asp Ile Pro Asn
   805 810 815
   Val Gly Lys Asn Ala Leu Arg Asn Leu Asp Glu Arg Gly Ile Ile Arg
   820 825 830
   Ile Gly Ala Glu Val Lys Asp Gly Asp Ile Leu Val Gly Lys Val Thr
   835 840 845
   Pro Lys Gly Val Thr Glu Leu Thr Ala Glu Glu Arg Leu Leu His Ala
   850 855 860
   Ile Phe Gly Glu Lys Ala Arg Glu Val Arg Asp Thr Ser Leu Arg Ala
   865 870 875 880
   Pro His Gly Gly Asp Gly Ile Val Leu Asp Val Lys Ile Phe Asn Arg
   885 890 895
   Glu Asp Gly Asp Glu Leu Pro Pro Gly Val Asn Gln Leu Val Arg Val
   900 905 910
   Tyr Ile Val Gln Lys Arg Lys Ile Asn Gln Gly Asp Lys Met Ala Gly
   915 920 925
   Arg His Gly Asn Lys Gly Val Ile Ser Arg Ile Leu Pro Glu Glu Asp
   930 935 940
   Met Pro Phe Leu Pro Asp Gly Thr Pro Val Asp Ile Met Leu Asn Pro
   945 950 955 960
   Leu Gly Val Pro Ser Arg Met Asn Ile Gly Gln Val Leu Glu Leu His
   965 970 975
   Leu Gly Met Ala Ala Arg Lys Leu Gly Ile His Val Ala Ser Pro Val
   980 985 990
   Phe Asp Gly Ala Ser Glu Glu Asp Val Trp Gly Thr Leu Glu Glu Ala
   995 1000 1005
   Gly Met Ala Arg Asp Gly Lys Thr Ile Leu Tyr Asp Gly Arg Thr
   1010 1015 1020
   Gly Glu Pro Phe Asp Asn Arg Val Ser Val Gly Ile Met Tyr Met
   1025 1030 1035
   Ile Lys Leu Ala His Met Val Glu Arg Pro Ser Ser Met Arg Val
   1040 1045 1050
   Asn Arg Pro Val Thr His Ser Leu Thr Gln Gln Leu Leu Gly Gly
   1055 1060 1065
   Lys Ala Gln Phe Gly Gly Gln Arg Phe Gly Glu Met Glu Val Trp
   1070 1075 1080
   Ala Leu Glu Ala Tyr Gly Ala Ala Tyr Thr Leu Pro Arg Asn Pro
   1085 1090 1095
   Ser Leu Leu Asn Ser Asp Asp Val Val Gly Arg Val Lys Thr Tyr
   1100 1105 1110
   Glu Ala Ile Val Lys Gly Glu Asn Val Pro Glu Pro Gly Val Pro
   1115 1120 1125
   Glu Ser Phe Lys Val Leu Ile Lys Glu Leu Gln Ser Leu Gly Met
   1130 1135 1140
   Asp Val Lys Met Leu Ser Ser Asn Glu Glu Glu Ile Glu Met Arg
   1145 1150 1155
   Glu Leu Asp Asp Glu Glu Asp Gln Thr Ser Glu Lys Leu Asn Leu
   1160 1165 1170
   Asn Leu Glu Thr Asn Glu Ser Gln Leu
   1175 1180